# EUROPEAN PATENT APPLICATION

(11) **EP 1 674 564 A1**
(43) Date of publication of application: **28.06.2006**
(21) Application number: 04748306.0
(22) Date of filing: 06.08.2004
(51) Int. Cl.: C12N 9/02, C12N 15/53, C12N 5/10, C12Q 1/02, C07K 16/18, C12Q 1/68, C07K 16/40, C07K 16/46, C12P 21/08, A61K 31/7105, A61K 35/12, A61K 39/395, A61K 48/00, A61P 35/00, G01N 33/15, G01N 33/50, G01N 33/53, G01N 33/566

(54) **ANTIBODY AGAINST NOX1 POLYPEPTIDE, METHOD OF DIAGNOSING CANCER WITH THE USE OF NOX1 GENE AND METHOD OF SCREENING CANCER GROWTH INHIBITOR**

(30) Priority: 01.09.2003 JP 2003308658
(71) Applicant: Kureha Corporation, Tokyo 103-8552 (JP); Mitsushita, Junji, Nagano 390-0802 (JP); Kamata, Tohru, Nagano 390-0804 (JP)
(72) Inventor: MITSUSHITA, Junji, Matsumoto-shi, Nagano 3900802 (JP); KAMATA, Tohru, Matsumoto-shi, Nagano 3900804 (JP); HIROSE, Kunitaka, Tokyo 1790074 (JP)
(74) Representative: Jones, Helen M.M.
(86) International application number: PCT/JP2004/011673
(87) International publication number: WO 2005/021739

(57) **Abstract**

The present invention provides a diagnostic method for cancer, a screening method for a cancer growth inhibitor, and a pharmaceutical composition used in cancer therapy using a Nox1 gene associated with a mutant Ras oncogene. More specifically, the present invention relates to: a composition for producing an antibody, comprising a polypeptide coded for a Nox1 gene, a homologue thereof, and their peptide fragments; an antibody against the polypeptide coded for a Nox1 gene; and a method for detecting the antibody or Nox1 -expressing mRNA.

## Description

### Technical Field

The present invention relates to a diagnostic method for cancer, a screening method for a cancer growth inhibitor, and a pharmaceutical composition based on a cancer-associated gene and a polypeptide coded for the gene. More specifically, the present invention relates to a diagnostic method for cancer; a screening method for a cancer growth inhibitor; and a pharmaceutical composition used in cancer therapy, using a Nox1 gene associated with a mutant Ras oncogene, a polypeptide coded for the gene, and an antibody specific to the polypeptide.

### Background Art

A Mutant Ras oncogene is heretofore known to exert great influence on mammalian cell carcinogenesis and its progression (Non-Patent Document 1). For example, the mutant Ras oncogene is considered to cancerate animal cells and aggravate the cancer via a Raf-MAPKK-MAPK pathway, one of its principal downstream pathways (Non-Patent Document 2).

Based on these findings, there have been developed a method for tumor suppressor gene therapy comprising administering an expression vector gene comprising a gene coding for p94RB to a targeted cancer cell depleted of N-ras tumor suppressor (Patent Document 1), and a method of identifying a tumor suppressor gene by use of H-ras, K-ras, and N-ras oncogenes (Patent Document 2).

Recent research has reported that cells transformed with mutant Ras gene produce reactive oxygen species (ROS) such as superoxide and H₂O₂ (Non-Patent Document 3). Low-level intracellular ROS plays a role as a signal molecule in growth factor-induced cell growth (Non-Patent Documents 4 and 5), suggesting that a rise in ROS generation associated with mutant Ras gene possibly causes the abnormal growth of animal cells.

However, reactive oxygen-producing enzymes that are involved in carcinogenesis, cancer promotion, and so on, caused by mutant Ras gene have heretofore been unknown.
(Patent Document 1) National Publication of International Patent Application No. 1996-508166
(Patent Document 2) National Publication of International Patent Application No. 1998-504448
(Non-Patent Document 1) Lowy, D. R. Annu. Rev. Biochem. 62, 851-891 (1993)
(Non-Patent Document 2) McCormick, F. TCB. 9, M53-M56 (1999)
(Non-Patent Document 3) Irani, K. et al. Science 275, 1649-1652 (1997)
(Non-Patent Document 4) Sundaresan, M. et al. Science. 270, 296-299 (1995)
(Non-Patent Document 5) Bae, Y. S. et al. J. Biol. Chem. 272, 217-221 (1997)

### Disclosure of the Invention

An object of the present invention is to provide a diagnostic method for cancer and a screening method for a cancer growth inhibitor; and a pharmaceutical composition used in cancer therapy using a Nox-1 gene associated with a mutant Ras oncogene, a polypeptide coded by the gene, and an antibody specific to the polypeptide.

The present inventors conducted studies and consequently gained findings that a mutant Ras oncogene remarkably increases the expression of Nox1 (1, 2, and 3), a homologue of the catalytic subunit of superoxide-generating NADPH oxidase, in an MAPKK-MAPK pathway, and that siRNA of the Nox1 gene effectively suppresses phenotypes of a mutant Ras gene such as cell adhesion-dependent growth, morphological changes, and tumor formulation in athymic mice. Based on the findings, the present inventors attained the object and completed the present invention.

Namely, the present invention provides a composition for producing an antibody, comprising: (1) a polypeptide comprising the amino acid sequence of SEQ ID NO: 2; (2) a polypeptide having an amino acid sequence mutated from the amino acid sequence of SEQ ID NO: 2 by the substitution, deletion, addition, and/or insertion of one or more amino acid residues and inducing the production of an antibody specific to the polypeptide comprising the amino acid sequence of SEQ ID NO: 2; or (3) a polypeptide fragment having a partial sequence of the polypeptide of (1) or (2) and inducing the production of an antibody specific to the polypeptide comprising the amino acid sequence of SEQ ID NO: 2.

Moreover, the present invention provides a method for producing an antibody specific to a polypeptide comprising the amino acid sequence of SEQ ID NO: 2, comprising administering the composition for producing an antibody to a mammal.

Furthermore, the present invention provides an antibody specific to a polypeptide comprising the amino acid sequence of SEQ ID NO: 2.

Furthermore, the present invention provides a diagnostic method for cancer, comprising bringing the antibody specific to a polypeptide comprising the amino acid sequence of SEQ ID NO: 2 into contact with a biological sample.

Furthermore, the present invention provides a diagnostic composition for cancer and a pharmaceutical composition for cancer therapy, comprising the antibody.

Furthermore, the present invention provides a diagnostic method for cancer, characterized by detecting a polynucleotide comprising the nucleotide sequence of SEQ ID NO: 1 or a fragment thereof. It is preferred that the detection should be performed by detecting the polynucleotide or the fragment thereof by polymerase chain reaction or real-time quantitative polymerase chain reaction.

Furthermore, the present invention provides siRNA corresponding to a polynucleotide comprising the nucleotide sequence of SEQ ID NO: 1 or a fragment thereof.

Furthermore, the present invention provides a pharmaceutical composition for cancer therapy, comprising: siRNA corresponding to a polynucleotide comprising the nucleotide sequence of SEQ ID NO: 1 or a fragment thereof; and siRNA corresponding to a polynucleotide comprising a nucleotide sequence at positions from 71 to 1615 of SEQ ID NO: 1 or a fragment thereof.

Furthermore, the present invention provides a pharmaceutical composition for cancer therapy, comprising a cell transformed with siRNA corresponding to a polynucleotide comprising the nucleotide sequence of SEQ ID NO: 1 or a fragment thereof; and siRNA corresponding to a polynucleotide comprising a nucleotide sequence at positions from 71 to 1615 of SEQ ID NO: 1 or a fragment thereof.

Furthermore, the present invention provides a screening method for a cancer cell growth inhibitor targeted to a Nox1 gene, comprising: (1) transfecting a cell with a Nox1 gene; bringing the transformed cell into contact with a substance to be screened; and detecting the inactivation of Nox1 activity (primary screening); and (2) investigating whether or not the Nox1-inactivating substance suppresses cancer cell growth (secondary screening), thereby detecting the expression of the Nox1 gene and the inactivation of Nox1 activity.

### (Definition)

The term "polypeptide" used herein means a primary structure of an amino acid sequence, a polypeptide fragment with the primary structure, a polypeptide having biological activity accompanied with a three-dimensional structure, or a protein.

A "Nox1 polypeptide" used herein refers to a polypeptide coded for a Nox1 gene and includes both full-length polypeptide and polypeptide fragment.

The term "homologue" used herein refers to a polypeptide having homology in an amino acid sequence to a polypeptide or protein with a particular amino acid sequence and having biological activity or antigenicity in common with the polypeptide or protein.

The term "siRNA" used herein means a short RNA fragment that suppresses the expression of a particular gene or a double-stranded RNA molecule that consists of the RNA fragment and its complementary strand.

A "mutant Ras gene" used herein refers to a human Ras gene in a mutated state that is involved in tumor formation. The mutant Ras gene is, for example, a human Ras gene having a K-Ras mutation of glycine at the 12th codon to aspartic acid or to valine or arginines, which is commonly seen in human pancreatic cancer, as well as a human Ras gene having K-Ras mutations occurring at the 12th codon and additionally at the 13th and 61st codons, which are found in lung cancer.

A "partial sequence of an amino acid sequence" of the polypeptide used herein refers to a sequence that comprises at least 8 or more consecutive amino acid residues contained in the amino acid sequence.

An "oligonucleotide" used herein refers to a nucleotide that typically comprises less than 100 bases and preferably comprises 6 to 99 bases.

### Brief Description of the Drawings

Fig. 1(a) is a graph showing a result of gene expression analysis conducted by real-time quantitative polymerase chain reaction in Example 1;
Fig. 1(b), (c), and (d) are, results of detecting Nox1 expression in Example 2 by conducting real-time quantitative polymerase chain reaction in the same way as in Example 1;
Fig. 2 is an electrophoretic image obtained in Example 4 by conducting PCR by use of M13F and 3.0Rev as primers to confirm a result of transformation of cell lines stably transfected with RNAi(1), RNAi(3), or RNAi(5);
Fig. 3 shows photographs obtained in Example 4, wherein morphological changes in which Nox1 gene is involved are compared among cells;
Fig. 4 is a graph showing a result of measuring the adhesion-dependent cell growth of cell lines exhibited by soft agar culture in Example 4 in order to examine the transformation of cells by observing anchorage-independent growth capacity;
Fig. 5 is a graph showing a growth curve obtained in Example 4 by measuring the number of each of K-Ras-NRK/neg-1 (neg-1), K-Ras-NRK/RNAi(1)-7 (i(1)-7), K-Ras-NRK/RNAi(3)-19 (i(3)-19), and K-Ras-NRK/RNAi(5)-7 (i(5)-7) cells in a liquid medium;
Fig. 6 is a graph showing a result of quantifying a decrease in NBT in Example 5 by solubilizing treated cells and measuring absorbance at 510 nm;
Fig. 7 and Fig. 8 (left panel) show results from Example 6 that a GFP-rat Nox1 fusion polypeptide is produced, that the coexpression of RNAi(1), RNAi (3), and RNAi(5) inhibits the production of a GFP-rat Nox1 fusion protein dose-dependently on each vector (Fig. 7), and that the RNAi constructs have high specificity to their target gene (Fig. 8, left panel);
Fig. 8 (right panel) is an electrophoretic image obtained in Example 6 by conducting RT-PCR to confirm that the mRNA expression of endogenous Nox1 is inhibited in each of cells, as compared to K-Ras-NRK and K-Ras-NRK/neg-1 cells;
Fig. 9 is an image obtained in Example 7 by immunoblotting by the same approach as in Example 6, wherein the image demonstrates that siRNA used does not inhibit GFP or GFP-human Nox 1 expression;
Fig. 10 is a photograph showing the influence of reactivation of Nox1 once inhibited by siRNA on cells in terms of morphological changes in K-Ras-NRK cells in Example 7;
Fig. 11 is a graph showing the influence of reactivation of Nox1 once inhibited by siRNA on cells in terms of cell growth rates in Example 7;
Fig. 12 is a graph showing a result of investigating adhesion-dependent cell growth capacity by use of a soft agar culture method in order to examine the influence of reactivation of Nox1 once inhibited by siRNA on cells by observing anchorage-independent growth capacity in Example 7;
Fig. 13 is a photograph showing a result of observing morphological changes in cells in order to investigate the influence of Nox1 gene expression on transformation with mutant Ras in Example 8; and
Fig. 14 is a graph showing the influence of siRNA on tumor formation caused by mutant Ras by observing tumor formation in a nude mouse over 1 month and measuring the volume of the formed tumor in Example 9.

### Most Preferable Mode for Carrying Out the Invention

A composition for producing an antibody of the present invention comprises: (1) a polypeptide comprising the amino acid sequence of SEQ ID NO: 2; (2) a polypeptide having an amino acid sequence mutated from the amino acid sequence of SEQ ID NO: 2 by the substitution, deletion, addition, and/or insertion of one or more amino acid residues and inducing the production of an antibody specific to the polypeptide comprising the amino acid sequence of SEQ ID NO: 2; or (3) a polypeptide fragment having a partial sequence of the polypeptide of (1) or (2) and inducing the production of an antibody specific to the polypeptide comprising the amino acid sequence of SEQ ID NO: 2.

The composition for producing an antibody of the present invention may contain a polypeptide having at least 80% homology, preferably 90% homology, particularly preferably 95% homology, to the amino acid sequence of SEQ ID NO: 2. The homology of amino acid sequences used herein refers to identity (%) between a reference amino acid sequence and an amino acid sequence compared therewith when the sequences are aligned. The homology can be calculated using default (initial setting) parameters in analytical software such as BLAST (J. Mol. Biol., 215, 403 (1990)) and FASTA (Methods in Enzymology, 183, 63-69) that are standard programs for performing the homology search of nucleotide sequences and amino acid sequences.

The polypeptide comprising the amino acid sequence of SEQ ID NO: 2 is a polypeptide coded for a Nox1 gene. The carcinogenesis and cancer progression of tissue to be examined can be screened by detecting the production of the polypeptide. Although an antibody manufactured with the composition is specific to a human Nox1 polypeptide, the antibody can also be used in the screening of the carcinogenesis and cancer progression of non-human animals when having specificity to homologues of the polypeptide produced by the non-human animals. Thus, the composition for producing an antibody can also be used for producing an antibody for detecting a human cancer cell and an antibody for screening the carcinogenesis and cancer progression of mammals capable of producing human Nox1 polypeptide homologues.

The partial sequence of the amino acid sequence of SEQ ID NO: 2 is a sequence that comprises at least 8 or more consecutive amino acid residues contained in the amino acid sequence and refers to any of those serving as an epitope that induces the production of an antibody specific to the polypeptide comprising the amino acid sequence of SEQ ID NO: 2. When the partial sequence is used as an epitope, it can be used in combination with an appropriate carrier protein.

The polypeptide fragment of the present invention is any fragment of the polypeptides (1) and (2) and induces the production of an antibody specific to the polypeptide comprising the amino acid sequence of SEQ ID NO: 2.

The length of the peptide fragment is not particularly limited and however, is typically 20 to 200 amino acid residues, preferably 20 to 100 amino acid residues, more preferably 20 to 70 amino acid residues, in length.

In the present specification, the N-terminuses (amino terminuses) of the polypeptide, the homologue, and the polypeptide fragment (hereinafter, abbreviated to the polypeptide, if necessary) are indicated in the left, and the C terminuses (carboxyl terminuses) thereof are indicated in the right, in accordance with the standard notation. The polypeptide and so on, of the present invention can have a carboxyl group (-COOH), carboxylate (-COO-), amide (-CONH₂), or ester (-COOR) at the C terminus. Examples of the side chain R of this ester include: C₁ to C₆ alkyl groups such as methyl, ethyl, n-propyl, isopropyl, and n-hutyl; C₃ to C₈ cycloalkyl groups such as cyclopentyl and cyclohexyl; C₆ to C₁₂ aryl groups such as phenyl and α-naphthyl; and alkyl groups such as phenyl-C₁ to -C₂ alkyl groups such as benzyl and phenethyl or α-naphthyl-C₁ to -C₂ alkyl groups such as α-naphthylmethyl.

The polypeptide and so on, of the present invention also includes: those in which an amino group of an N-terminal amino acid residue is protected with a protecting group such as a formyl group and an acetyl group; those in which an N-terminal glutamine residue generated in vivo by cleavage is converted to pyroglutamic acid; those in which a substituent (e.g., -OH, -SH, an amino group, an imidazole group, an indole group, or a guanidino group) on the side chain of an intramolecular amino acid is protected with a suitable protecting group; or a conjugated protein such as so-called glycoprotein bound with a sugar chain.

Moreover, the polypeptide and so on, of the present invention can be used as a physiologically acceptable inorganic or organic acid-addition salt. Examples of the inorganic acid-addition salt include salts of hydrochloric acid, phosphoric acid, hydrobromic acid, and sulfuric acid, and examples of the organic acid-addition salt include salts of acetic acid, formic acid, propionic acid, fumaric acid, maleic acid, succinic acid, tartaric acid, citric acid, malic acid, oxalic acid, benzoic acid, methanesulfonic acid, and benzenesulfonic acid.

Namely, the composition for producing an antibody of the present invention comprises the polypeptide, the homologue thereof, and their peptide fragments, or their acid-addition salts. The composition may optionally contain ingredients such as a vehicle, a diluent, and an adjuvant.

The present invention provides a method of producing an antibody specific to the polypeptide comprising the amino acid sequence of SEQ ID NO: 2, the homologue thereof, or the peptide fragment by use of the composition for producing an antibody.

An antibody manufactured by the method of the present invention is not particularly limited as long as it can be used according to the object of the present invention. Examples of the antibody include a human/mouse chimeric antibody, a humanized antibody, or a human antibody. In this context, the humanized antibody refers to any of those containing several percent of a mouse-derived antibody in the whole antibody, while the human antibody refers to an antibody derived 100% from a human. Meanwhile, the chimeric human antibody refers to any of those containing 10 to 20% of a mouse-derived antibody. The antibody may be any of polyclonal and monoclonal antibodies.

The monoclonal antibody of the present invention can be manufactured by the following procedures: at first, the composition for producing an antibody is administered to non-human mammals. A complete Freund's adjuvant or incomplete Freund's adjuvant may be administered for enhancing the ability of the mammals to produce antibodies. The administration is typically performed once every 2 to 6 weeks with a total of approximately 2 to 10 doses. Examples of the non-human mammals include monkeys, rabbits, dogs, guinea pigs, mice, rats, sheep, goats, and chickens, with mice and rats generally preferred.

An individual in which antibody production has been observed is selected from the non-human mammals immunized with the composition. Two to Five days after final immunization, antibody-producing cells contained in the spleen or lymph node are collected from the individual and fused with myeloma cells from animals of the same or different species to thereby create monoclonal antibody-producing hybridomas. Antibody titer in antiserum is measured by allowing a labeled protein to react with the antiserum and then measuring the activity of the labeling agent bound with the antibody. Cell fusion can be performed by a routine method such as the method of Kohler and Milstein (Nature, 256, 495, 1975). For example, polyethylene glycol (PEG) or a Sendai virus can be used as a fusion promoter.

Examples of the myeloma cells include non-human mammalian myeloma cells such as NS-1, P3U1, SP2/O, and AP-1, with P3U1 particularly preferred. A preferable ratio of the number of the antibody-producing cells (spleen cells) to the number of the myeloma cells, used in the fusion, is approximately 1:1 to 20. The spleen and myeloma cells can be fused by incubation at 20 to 40°C, preferably 30 to 37°C, for 1 to 10 minutes, with PEG (preferably PEG1000 to PEG6000) added at a concentration of approximately 10 to 80%.

The monoclonal antibody-producing hybridomas can be screened by a variety of methods. Examples of the methods include: a method that involves bringing a hybridoma culture supernatant into contact with a solid phase (e.g., a microplate) where an antigen is adsorbed either directly or in combination with a carrier and subsequently bringing a solution containing an anti-immunoglobulin antibody labeled with a radioactive substance, an enzyme, or the like, into contact therewith to detect the monoclonal antibody bound to the solid phase; and a method that involves bringing a hybridoma culture supernatant into contact with a solid phase where an anti-immunoglobulin antibody or the like is adsorbed and subsequently bringing a solution of a protein labeled with a radioactive substance, an enzyme, or the like, into contact therewith to detect the monoclonal antibody bound to the solid phase.

The monoclonal antibody-producing hybridomas can be selected by a routine method. For example, a medium for animal cells supplemented with HAT (hypoxanthine-aminopterin- thymidine), an RPMI 1640 medium containing 10 to 20% fetal bovine serum, a GIT medium containing 1 to 10% fetal bovine serum (Wako Pure Chemical Industries), or a serum-free medium for culturing hybridomas (SFM-101; Nissui Pharmaceutical) can be used. A culture temperature is 20 to 40°C, and a culture time is typically 5 days to 3 weeks. The culture can typically be performed under 5% carbonic acid gas. Antibody titer in the hybridoma culture supernatant can be measured in the same way as in the above-described measurement of antibody titer in antiserum.

The obtained monoclonal antibody can be separated and purified by a routine method such as: an immunoglobulin separation and purification method such as salting-out, alcohol precipitation, isoelectric precipitation, and electrophoresis; an absorption and desorption method with an ion exchanger (DEAE); an ultracentrifugation method; a gel filtration method; an antigen-bound solid phase; or a specific purification method performed by collecting only an antibody with a protein A active adsorbent and then dissociating the bond to obtain the antibody.

Alternatively, the polyclonal antibody of the present invention can be manufactured by immunizing non-human mammals with the polypeptide and so on serving as an immunizing antigen or a complex of the peptide fragment thereof with a carrier protein and then collecting an antibody-containing component such as serum therefrom, followed by antibody separation and purification.

The mixing ratio of the immunizing antigen to the carrier protein is determined so that the antibody can efficiently be raised against the hapten that has been crosslinked with the carrier and used in immunization. For example, approximately 0.1 to 20 parts by weight, preferably approximately 1 to 5 parts by weight, of bovine serum albumin, bovine thyroglobulin, or hemocyanin can be used with respect to 1 part by weight of the hapten. For example, glutaraldehyde, carbodiimide, maleimide- activated ester, and activated ester reagents containing a thiol or dithiopyridyl group can be employed in the coupling between the hapten and the carrier.

The complex antigen may be administered alone or in combination with a carrier or diluent, or alternatively, for enhancing the ability of the mammals to produce antibodies, with a complete Freund's adjuvant or incomplete Freund's adjuvant. The administration is typically performed once every 2 to 6 weeks with a total of approximately 3 to 10 doses. The polyclonal antibody is collected from the blood, ascites, yolk, or the like, of the immunized mammal. Polyclonal antibody titer in antiserum can be measured in the same way as in the above-described measurement of antibody titer in antiserum. The polyclonal antibody can be separated and purified in the same way as in the above-described separation and purification of the monoclonal antibody.

A diagnostic kit for cancer of the present invention comprises the polyclonal or monoclonal antibody thus obtained. The diagnostic kit optionally contains wells for immune reaction, a staining agent, an enzyme-labeled antibody for detection, a cleansing liquid, an antibody diluent, a sample diluent, an enzyme substrate, a diluent of an enzyme substrate solution, and additional reagents.

A pharmaceutical composition comprising the antibody of the present invention suppresses the expression of a specific protein coded for a Nox1 gene and as such, can be employed in the prevention, delayed progression, and treatment of carcinogenesis.

The antibody can be administered orally or parenterally, and the parenteral administration includes local administration to tissue.

When the pharmaceutical composition of the present invention is orally administered, pharmaceutical ingredients such as carriers widely used, for example, fillers, expanders, binders, disintegrants, disintegration inhibitors, buffers, tonicity agents, emulsifiers, dispersants, stabilizers, coating agents, surfactants, absorption promoters, humectants, wetting agents, adsorbents, lubricants, and excipients can be used. Additives such as coloring agents, preservatives, perfumes, flavoring agents, and sweetening agents may optionally be used.

Concrete examples of the pharmaceutical ingredients include: excipients such as milk sugar, white sugar, sodium chloride, grape sugar, urea, starch, calcium carbonate, kaoline, crystalline cellulose, and silicic acid; binders such as water, ethanol, simple syrup, grape sugar fluids, starch fluids, gelatin solutions, carboxymethylcellulose, shellac, methylcellulose, potassium phosphate, and polyvinylpyrrolidone; disintegrants such as dried starch, sodium alginate, agar powder, laminaran powder, sodium hydrogencarbonate, calcium carbonate, polyoxyethylene sorbitan fatty acid ester, sodium lauryl sulfate, stearic acid monoglyceride, starch, and milk sugar; disintegration inhibitors such as white sugar, stearic acid, cacao butter, and hydrogenated oil; absorption promoters such as quaternary ammonium salts and sodium lauryl sulfate; humectants such as glycerin and starch; adsorbents such as starch, milk sugar, kaolin, bentonite, and colloidal silicic acid; and lubricants such as purified talc and stearate. In addition, the technique of a drug delivery system known in the art is optionally adopted for each of the dosage forms to provide for the sustained release, local application (e.g., troches, buccals, and sublingual tablets), drug release control, conversion to an enteric coated form, conversion to a gastrolytic form, and so on, of the pharmaceutical composition.

When the pharmaceutical composition of the present invention is parenterally administered, the pharmaceutical composition can be used in forms such as administration by injection such as instillation, intravenous injection, hypodermic injection, and intramuscular injection, and endorectal administration with suppositories, for example, suppositories made of fats and oils and water-soluble suppositories. The pharmaceutical composition in such a form can be prepared with ease by a routine method using a typical carrier in the pharmaceutical field.

The pharmaceutical composition comprising the antibody of the present invention can be administered to, for example, humans or other mammals (e.g., rats, mice, guinea pigs, rabbits, sheep, pigs, cattle, horses, cats, dogs, and monkeys). The amount of the pharmaceutical composition administered appropriately differs depending on the conditions of objects to be administered, administration routes, and so on. For example, when orally administered, the pharmaceutical composition is generally administered at approximately 10 to 4000 mg, preferably approximately 20 to 2000 mg, more preferably approximately 50 to 500 mg, per day for an adult patient weighing 60 kg. When parenterally administered, it is preferred that the composition, for example in the form of injection, should be administered through the vein at a dose of approximately 10 to 2000 mg, preferably approximately 20 to 1000 mg, more preferably approximately 50 to 500 mg, per day for an adult patient weighing 60 kg although the dose differs depending on objects to be administered, the condition of the target cancer, and so on.

Cancer diagnosis can be performed by detecting and quantifying a Nox1 gene in biological tissue or bodily fluids by immunochemical assay comprising bringing the antibody of the present invention into contact with a biological sample.

When this immunochemical assay is conducted, the antibody of the present invention is held in a carrier. Examples of the carrier used in the assay include: gel particles such as agarose gels (e.g., Sepharose 4B and Sepharose 6B; Pharmacia Fine Chemicals), dextran gels (e.g., Sephadex G-75, Sephadex G-100, and Sephadex G-200; Pharmacia Fine Chemicals), polyacrylamide gels (Bio-Gel P-30, Bio-Gel P-60, and Bio-Gel P-100; BioRad Laboratories), and cellulose particles (Avicel; Asahi Kasei); ion-exchange cellulose such as diethylaminoethylcellulose and carboxymethylcellulose; physical adsorbents such as glass, silicone strips, and stainless steel-based resins; immunoassay plates (Nunc); and weakly basic anion-exchange resins (e.g., Amberlite IR-4B and DOWEX-3; Dow Chemical).

The antibody can be held in the carrier by a routine method such as cyanogen bromide and glutaraldehyde methods. In a more convenient way, the carrier may be adsorbed physically onto the surface of the antibody. Examples of a labeling agent to be bound to the antibody include radioactive isotopes, enzymes, fluorescent substances, and luminescent substances, with enzymes preferably used.

Any of those being stable and having high specific activity is preferable as the enzyme, and peroxidase, alkaline phosphatase, β-D-galactosidase, and glucose oxidase can be used, with peroxidase particularly preferred.

A sample to be tested in the immunochemical assay system of the present invention is a bodily fluid such as urine, serum, plasma, and spinal fluid, or an animal cell or a culture supernatant thereof. When the immunochemical assay of the present invention is conducted, a substance to be analyzed such as a Nox1 polypeptide to be measured is added to the antibody held in the carrier to perform antigen-antibody reaction. Then, the bound substance of an anti-Nox1 antibody and the labeling agent is further added thereto and reacted. The resulting reaction product is supplemented with the labeling agent (e.g., substrate of enzyme). The enzyme activity of the reaction product can be determined by measuring the absorbance or fluorescence intensity of a generated substance. These procedures are performed in advance on a standard solution of a known amount of the labeling agent (e.g., enzyme) to construct a standard curve that plots the relationship of absorbance or fluorescence intensity versus the amount of the Nox1 polypeptide or the like. This standard curve is compared with the absorbance or fluorescence intensity obtained from the substance to be analyzed (sample to be tested) comprising an unknown amount of the Nox1 polypeptide to measure the amount of the Nox1 polypeptide in the substance to be analyzed.

Moreover, the present invention provides a diagnostic method for cancer, characterized by detecting a polynucleotide comprising the nucleotide sequence of SEQ ID NO: 1 or a fragment thereof. The polynucleotide or the fragment thereof can be detected by a routine method utilizing polymerase chain reaction (PCR) or real-time quantitative polymerase chain reaction.

The polymerase chain reaction or the real-time quantitative polymerase chain reaction uses a sense strand fragment corresponding to the nucleotide sequence of SEQ ID NO: 1 as a forward primer and an antisense strand fragment corresponding to the nucleotide sequence of SEQ ID NO: 1 as a reverse primer. The length of the forward primer dose not have to be particularly limited and however, is typically 14 to 60 bases. Similarly, although the length of the reverse primer dose not have to be particularly limited, it is usually preferred that the length should be 14 to 60 bases. Examples of the forward and reverse primers used in the diagnostic method for cancer of the present invention include the followings:
the forward primer of 5'- GGAGCAGGAATTGGGGTCAC -3' (SEQ ID NO: 5); and
the reverse primer of 5'- TTGCTGTCCCATCCGGTGAG -3' (SEQ ID NO: 6).

When the detection is performed by the real-time quantitative polymerase chain reaction, examples of forward and reverse primers and a TaqMan probe used therein include the followings:
the forward primer of 5'- CCACTGTAGGCGCCCTAAGTT -3' (SEQ ID NO: 7);
the reverse primer of 5'- AAGAATGACCGGTGCAAGGA -3' (SEQ ID NO: 8); and
the TaqMan probe of 5'- AAGGGCATCCCCCTGAGTCTTGGAA -3' (SEQ ID NO: 9).

In the present invention, siRNA corresponding to a polynucleotide comprising the nucleotide sequence of SEQ ID NO: 1 or a fragment thereof suppresses Nox1 gene expression and reduces Nox1 polypeptide production. Thus, the use of the siRNA or oligonucleotide will allow the inhibition of cell carcinogenesis or cancer progression induced by a mutant Ras gene.

The siRNA or oligonucleotide of the present invention refers to a nucleotide or an oligonucleotide complementary to a polynucleotide comprising a consecutive nucleotide sequence of SEQ ID NO: 1 or a fragment thereof.

The siRNA of the present invention may correspond to a polynucleotide comprising a nucleotide sequence at positions from 71 to 1615 of SEQ ID NO: 1 or a fragment thereof. The length of the siRNA is less than 100 bp and is preferably 8 to 99 bp, particularly
preferably 10 to 30 bp. Moreover, preferable examples of the siRNA include the following:
siRNAs for human Nox1 of 
5'-GCGTGGCTTCAGCATGGAATTCAAGAGATTCCATGCTGAAGCCACGCTTTTT
TGGAAA-3' (SEQ ID NO: 10); and
 
5'-GGGCTTTCGAACAACAATATTCAAGAGATATTGTTGTTCGAAAGCCCTTTTT
TGGAAA-3' (SEQ ID NO: 11); and
siRNAs for rat Nox1 of

5'-GTTATGAGAAGTCTGACAAGTTCAAGAGACTTGTCAGACTTCTCATAATTTT
TTGGAAA-3' (SEQ ID NO: 12);

5'-GATTCTTGGCTAAATCCCATTCAAGAGATGGGATTTAGCCAAGAATCTTTTT
TGGAAA-3' (SEQ ID NO: 13); and

5'-GGACATTTGAACAACAGCATTCAAGAGATGCTGTTGTTCAAATGTCCTTTTT
TGGAAA-3' (SEQ ID NO: 14).

The siRNA of the present invention can be used by directly administrating the siRNA to a patient to be treated; by transfecting the siRNA into a cell taken out of a patient to be treated and then returning the cell to the patient; or by administering an expression vector incorporating the siRNA therein to a patient to be treated and expressing the siRNA.

Thus, the present invention provides a pharmaceutical composition for cancer therapy, comprising: siRNA corresponding to a polynucleotide comprising the nucleotide sequence of SEQ ID NO: or a fragment thereof; and siRNA corresponding to a polynucleotide comprising a nucleotide sequence at positions from 71 to 1615 of SEQ ID NO: 1 or a fragment thereof.

The pharmaceutical composition can be manufactured as the foregoing pharmaceutical composition comprising the antibody, by a routine method utilizing a pharmaceutical material conventionally used. The pharmaceutical composition comprising the siRNA of the present invention can be administered to, for example, humans or other mammals (e.g., rats, mice, guinea pigs, rabbits, sheep, pigs, cattle, horses, cats, dogs, and monkeys). The amount of the pharmaceutical composition administered appropriately differs depending on the conditions of objects to be administered, administration routes, and so on. For example, when orally administered, the pharmaceutical composition is generally administered at approximately 10 to 4000 mg, preferably approximately 20 to 2000 mg, more preferably approximately 50 to 500 mg, per day for an adult patient weighing 60 kg. When parenterally administered, it is preferred that the siRNA, for example in the form of injection, should be administered through the vein at a dose of approximately 10 to 2000 mg, preferably approximately 20 to 1000 mg, more preferably approximately 50 to 500 mg, per day for an adult patient weighing 60 kg although the dose differs depending on objects to be administered, the condition of cancer to be treated, and so on.

Moreover, the present invention provides a pharmaceutical composition for cancer therapy, comprising a cell transformed with siRNA corresponding to a polynucleotide comprising the nucleotide sequence of SEQ ID NO: 1 or a fragment thereof; and siRNA corresponding to a polynucleotide comprising a nucleotide sequence at positions from 71 to 1615 of SEQ ID NO: 1 or a fragment thereof.

In this context, the cell to be transformed may be a cell donated from a third party or a cell taken out of a patient to be treated. Alternatively, the transformed cell can be used as a cell for cancer therapy.

Moreover, the present invention provides an RNA molecule consisting of a partial sequence of the nucleotide sequence of SEQ ID NO: 1, or an RNA molecule consisting of a mutant nucleotide sequence of the partial sequence with the addition, deletion, or substitution of at least one base, and suppressing Nox1 polypeptide expression in a human cell. The RNA molecule (siRNA) can be designed on the basis of the sequence of the polynucleotide of the present invention according to a method known in the art (e.g., Nature, Vol. 411, pp. 494, 2001).

When the RNA molecule of the present invention is used in vivo or in vitro, it is used in the form of a double-stranded RNA molecule that consists of the RNA molecule and its complementary RNA. In this case, it is preferred that they should be treated so that the double-stranded RNA molecule is not degraded in a cell or is not dissociated into single strands. The treatment is performed by, for example, a method that adds a hydroxyl group to the 3' end of the RNA molecule, forms chemical bonds at both ends of the double strands through thiophosphoryl groups, or induces a chemical bond between both strands by ultraviolet radiation or through a bifunctional group such as bis(2-chloromethyl)amine, 4-thiouracil, or psoralen. The RNA molecule of the present invention suppresses Nox1 gene expression accompanying transformation with a mutant Ras gene and as such, can be used as a pharmaceutical composition for the prevention of cell carcinogenesis, the inhibition of cancer progression, and cancer therapy.

Moreover, the present invention provides a screening method for a cancer cell growth inhibitor targeted for a Nox1 gene. The screening method comprises bringing a cell having a mutant Ras gene or a normal cell transfected with a Nox1 gene into contact with a substance to be screened and then detecting the expression of the Nox1 gene and the inactivation of its enzyme activity. Subsequently, the transformed cell is cultured together with the substance to be screened to examine its ability to inhibit growth.

The influence of a compound to be screened on Nox1 gene expression can be examined by detecting an increase or decrease in mRNA expression by real-time quantitative polymerase chain reaction; by detecting a polypeptide or a peptide fragment thereof coded for the Nox1 gene with an antibody; or by observing morphological changes in the transformed cell.

The cell having a mutant Ras gene that can be used in the screening method, is not particularly limited, and examples thereof include H-Ras-NIH3T3 cells, K-Ras-NRK cells, and pancreatic cancer cells. For example, pEGFP-C1 (control) and pEGFP-C1-Nox1 can be used as a vector used in the introduction of the Nox1 gene, while for example, pSilencer can be used as an siRNA-expressing vector.

A cell line highly expressing Nox1 can be established by stably transfecting the pEGF-C1-Nox1 vector into an NIH3T3 or NRK cell by use of a Lipofectamine method. Alternatively, a pancreatic cancer cell having a mutant Ras gene can be utilized. It is preferred to use any of those separated and established from a human pancreatic cancer patient as the pancreatic cancer cell. These cells are cultured in a standard culture solution (Dulbecco's modified Eagle medium (DMEM) containing 10% fetal bovine serum (FBS)) in the presence of 5% CO₂. High-throughput screening can be conducted by culturing the cells in a multi-plate (96 or 48 wells), to which the substance to be screened is then added.

### (Example 1)

### Confirmation of Nox1 gene expression elevated by mutant Ras gene

Because Nox1 gene overexpression in NIH3T3 cells elevates superoxide generation and cell growth (1 and 2), the present inventors assumed that Nox1 is associated with the phenotypic expression of particular oncogenes. Therefore, cells transformed with K-Ras Val12 oncogene were used to confirm Nox1 gene expression elevated by Ras oncogene.

At first, rat kidney (NRK) cells and K-Ras-NRK cells (Kirstein murine sarcoma virus transformed NRK cells) were prepared. The K-Ras-NRK cells were purchased from ATCC.

The presence or absence of Nox1 expression in the rat kidney (NRK) cells and the K-Ras-NRK cells was initially detected by real-time quantitative polymerase chain reaction. The real-time quantitative polymerase chain reaction used a forward primer of SEQ ID NO: 15, a reverse primer of SEQ ID NO: 16, and a TaqMan MGB probe of SEQ ID NO: 17.
Forward Primer:
5'- GGTCACTCCCTTTGCTTCCA -3' (SEQ ID NO: 15)
Reverse Primer:
5'- GGCAAAGGCACCTGTCTCTCT -3' (SEQ ID NO: 16)
TaqMan MGB Probe:
5'- TCCAGTAGAAATAGATCTTT -3' (SEQ ID NO: 17)

The real-time quantitative polymerase chain reaction was performed using ABI Prism 7700 (Applied Biosystems) under reaction conditions set to defaults. Analytical values were standardized using rRNA 18S.

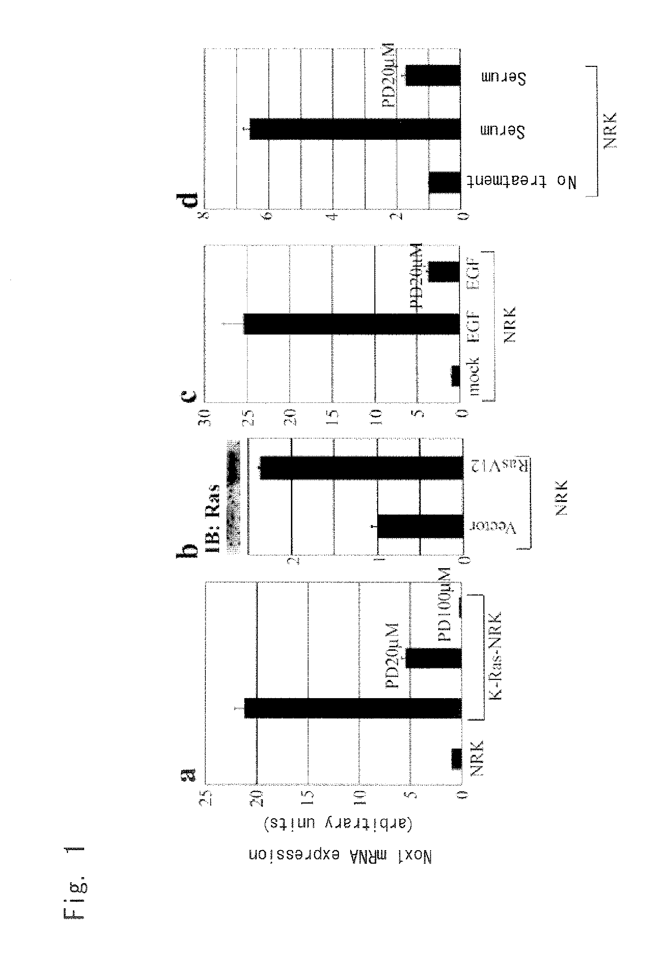

As a result of gene expression analysis by the real-time quantitative polymerase chain reaction, Nox1 gene expression was elevated in the K-Ras-NRK cells transformed with the K-Ras oncogene, as shown in the graph of Fig. 1(a).

### (Example 2)

### Confirmation of Nox1 gene expression elevated by transient transfection with mutant Ras gene

For analyzing the influence of transient transfection with mutant Ras gene, pcDNA3.1 vectors (blank vectors) and pcDNA3.1 carrying Ras Val12 vectors were used and transiently transfected into NRK cells. Forty-eight hours after the transfection, real-time quantitative polymerase chain reaction was conducted in the same way as in Example 1 in order to detect Nox1 expression. As a result, Nox1 gene expression was also elevated in the NRK cells transiently transfected with the H-Ras Val12, as compared with Nox1 gene expression in the control vector used, as shown in Fig. 1(b). The expression of the transfected Ras V-12 was confirmed by immunoblotting (IB) using a rabbit anti-Ras antibody.

For performing the immunoblotting, 1 x 10⁵ sample cells were solubilized with 2x sample buffers (0.1 M Tris-Cl (pH 6.8), 20% glycerol, 4% SDS, 3.1% DTT, and 0.001% BPB), and the polypeptides were separated by SDS gel electrophoresis and subsequently analyzed by the immunoblotting.

The proteins were electrically transferred to a nitrocellulose membrane in a transfer buffer (25 mM Tris-Cl (pH 8.3), 92 mM glycine, and 20% methanol) and reacted with an anti-Ras antibody (primary antibody) and an HRP-conjugated anti-rabbit-IgG antibody, followed by detection by a chemiluminescence (ECL) method.

As described above, the results of Examples 1 and 2 indicated that Nox1 gene expression is elevated by the action of mutant Ras oncogene.

NIH3T3 cells cultured overnight under serum-free conditions were treated mitogeniclally with serum (30%) or 50 ng/ml epidermal growth factor (EGF) and then analyzed by real-time quantitative polymerase chain reaction. As a result, Nox1 gene expression was increased to 6 to 20 times within 12 hours from the initiation of the culture, as shown in Figs. 1(c) and (d). That is, Nox1 gene expression is associated with factors that promote cell growth. This is consistent with the reports saying that both platelet-derived growth factor (PDGF) and angiotensin II induce superoxide formation and Nox1 gene expression in vascular smooth muscle cells (1 and 4).

### (Example 3)

### Analysis of signaling pathway of Nox1 gene expression caused by mutant Ras gene

A series of experiments were performed for analyzing the downstream region of a Ras signaling pathway of Nox1 gene expression elevated by mutant Ras gene. In Examples 1 and 2, the cells were treated for 12 hours with PD98059 (PD: 20 µM, 100 µM), an MAPKK inhibitor, to analyze Nox1 expression in the K-Ras-NRK cells and serum- and EGF-induced Nox1 expression by real-time quantitative polymerase chain reaction.

As a result, Nox1 expression in the K-Ras-NRK cells was inhibited by the PD98059 concentration-dependently (Fig. 1(a)). Moreover, serum- and EGF-induced Nox1 expression was respectively inhibited by 20 µM PD98059 (Figs. 1(c) and (d)).

On the other hand, wortmannin (100 nM), a P13 kinase inhibitor, did not inhibit an increase in Nox1 expression in the H-Ras-NIH3T3 cells in a control experiment (data not shown). These results indicated that the signals of mutant Ras gene and growth factors thereof induce Nox1 expression, by pathways not via P13K but via Ras-MAPKK-MAPK.

### (Example 4)

### Involvement of Nox1 gene in transformation with mutant Ras gene

Oligonucleotides 1-S and 1-AS were annealed and subcloned into a pSilencer hygro vector with an H1-promoter (Ambion) to yield RNAi(1). Likewise, oligonucleotides 3-S and 3-AS were annealed and subcloned into a pSilencer hygro vector with an H1-promoter (Ambion) to yield RNAi(3). In addition, oligonucleotides 5-S and 5-AS were annealed and subcloned into a pSilencer hygro vector with an H1-promoter (Ambion) to yield RNAi(5). A psilencer hygro Negative Control plasmid (Ambion) was used as a control vector.

The RNAi(1) is an siRNA construction targeted for positions from 223 to 241 of SEQ ID NO: 3. The RNAi(3) is an siRNA construction targeted for positions from 578 to 596 of SEQ ID NO: 3. The RNAi(5) is an siRNA construction targeted for positions from 1224 to 1242 of SEQ ID NO: 3.

The sequence of each oligonucleotide is as follows:

1-S:

5’-GATCCCGTTATGAGAAGTCTGACAAGTTCAAGAGACTTGTCAGACTTCTCAT

AATTTTTTGGAAA-3’ (SEQ ID NO: 18)

1-AS:
5'-AGCTTTTCCAAAAAATTATGAGAAGTCTGACAAGTCTCTTGAACTTGTCAGA
CTTCTCATAACGG-3' (SEQ ID NO: 19)

3-S:
5'-GATCCCGATTCTTGGCTAAATCCCATTCAAGAGATGGGATTTAGCCAAGAAT
CTTTTTTGGAAA-3' (SEQ ID NO: 20)

3-AS:
5'-AGCTTTTCCAAAAAAGATTCTTGGCTAAATCCCATCTCTTGAATGGGATTTA
GCCAAGAATCGG-3' (SEQ ID NO: 21)

5-S:
5'-GATCCCGGACATTTGAACAACAGCATTCAAGAGATGCTGTTGTTCAAATGTC
CTTTTTTGGAAA-3' (SEQ ID NO: 22)

5-AS:
5'-AGCTTTTCCAAAAAAGGACATTTGAACAACAGCATCTCTTGAATGCTGTTGT
TCAAATGTCCGG-3' (SEQ ID NO: 23)

4 µg each of the RNAi(1), RNAi(3), RNAi(5), and pSilencer hygro Negative Control plasmid was transfected respectively into 1x10⁶ K-Ras-NRK cells using Lipofectamine (Gibco-BRL). The transfected cells were cultured for selection at 37°C for 2 to 3 weeks in a DMEM medium supplemented with 10% fetal bovine serum (FBS) and 400 µg/ml hygromycin B under a 5% CO₂ moisture environment. After the culture, surviving colonies were isolated.

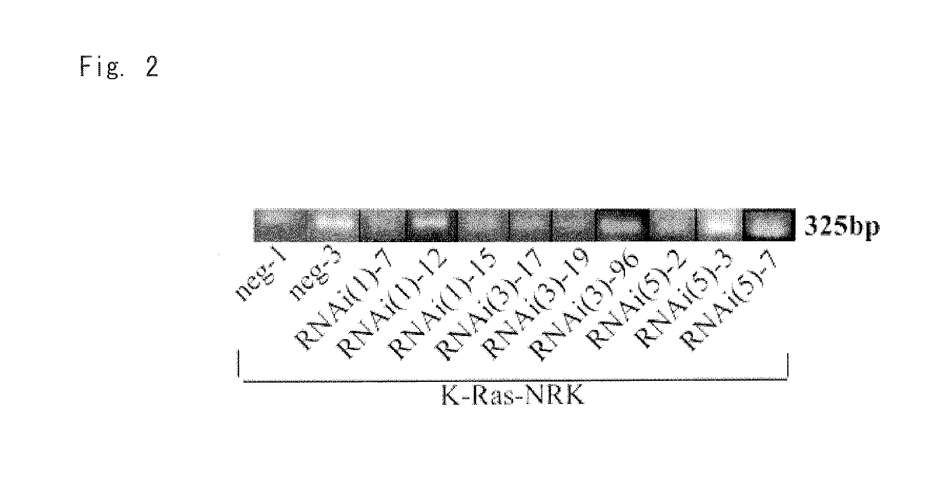

Three clones of the cell lines stably transfected with each of the RNAi(1), RNAi(3), and RNAi(5) (K-Ras-NRK/RRNAi(1)-7, K-Ras-NRK/RNAi(1)-12, and K-Ras-NRK/RNAi(1)-15; K-Ras-NRK/RNAi(3)-17, K-Ras-NRK/RNAi(3)-19, and K-Ras-NRK/RNAi(3)-96; and K-Ras-NRK/RNAi(5)-2, K-Ras-NRK/RNAi(5)-3, and K-Ras-NRK/RNAi(5)-7) as well as two clones of the cells stably transfected with the pSilencer hygro Negative Control plasmid (K-Ras-NRK/neg-1 and K-Ras-NRK/neg-3) were selected. The transfection of these constructions was confirmed by PCR using M13F and 3.0Rev as primers (Fig. 2). PCR temperature conditions were set to 94°C for 2 minutes, 30 cycles of (94°C for 1 minute, 60°C for 1 minute, and 72°C for 1 minute), followed by 72°C for 10 minutes. A thermal cycler used was Takara Thermal Cycler SP (Takara Shuzo Company Limited).
(Primer Sequence)
M13F: 5'- GTTTTCCCAGTCACGAC-3' (SEQ ID NO:24)
3.0Rev: 5'-GAGTTAGCTCACTCATTAGGC-3' (SEQ ID NO: 25)

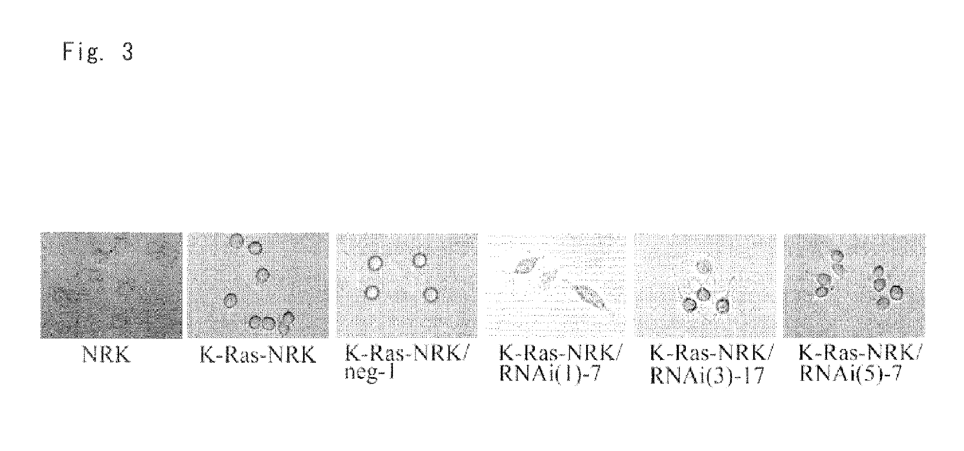

Next, celluler morphological changes in which Nox1 gene was involved were compared among the cells. As a result, as shown in Fig. 3, the K-Ras-NRK cells transfected with the RNAi(1) to RNAi(3) were extended, whereas the K-Ras-NRK cells transfected with the pSilencer hygro Negative Control plasmid morphologically remained round as the cells observed before introduction.

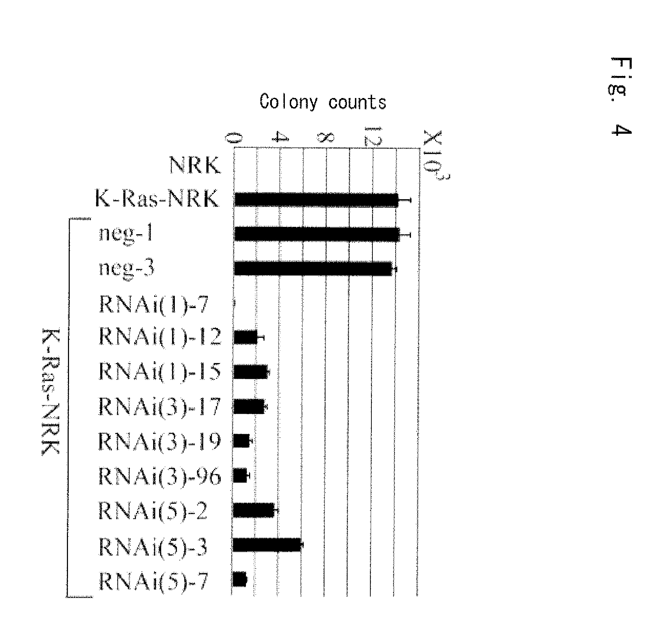

Subsequently, soft agar culture assay was performed in order to examine the transformation of the cells by observing anchorage-independent growth capacity. An agarose layer containing 0.53% agar and nutrients necessary for cell growth was placed and solidified in a culture dish of 6 cm in diameter, on which the cells suspended in a DMEM containing 0.3% agar and 10% FBS were then piled up at a final concentration of 1.5x10⁴ cells/culture dish. Subsequently, the cells were cultured at 37°C under a 5% CO₂ moisture environment to observe the appearance of cell colonies over 3 weeks. The adhesion-dependent cell growth of the cell lines exhibited by the soft agar culture was measured, and +/- average standard deviation (s.e.m.) from three measurements was shown in Fig. 4. As a result, the formation of many colonies was observed in the K-Ras-NRK cells and the transfected cells with the pSilencer hygro Negative Control plasmid (neg-1 and neg-3), whereas colony formation was remarkably inhibited in the transfection with the RNAi(1) to RNAi(5).

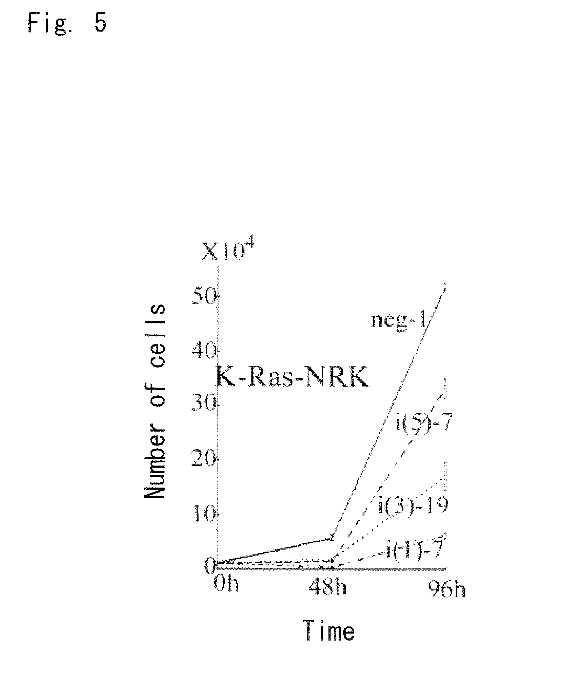

In addition, 10⁴ cells each of the K-Ras-NRK/neg-1 (neg-1), K-Ras-NRK/RNAi(1)-7 (i(1)-7), K-Ras-NRK/RNAi(3)-19 (i(3)-19), and K-Ras-NRK/i(5)-7 (i(5)-7) cells were transferred from the culture dish to a culture solution and then liquid-cultured for 6 days in a standard culture solution (DMEM containing 10% FBS) in the presence of 5% CO₂. Subsequently, the number of the cells in the liquid medium was measured, and its growth curve was shown in Fig. 5. As a result, each growth rate of the K-Ras-NRK/RNAi(1)-7, K-Ras-NRK/RNAi(3)-19, and K-Ras-NRK/RNAi(5)-7 cells was also decreased in the liquid culture, and by contrast, inhibitory effect on growth was not observed in the K-Ras-NRK/neg-1.

### (Example 5)

### Confirmation of superoxide production by NADPH oxidase coded for Nox1 gene

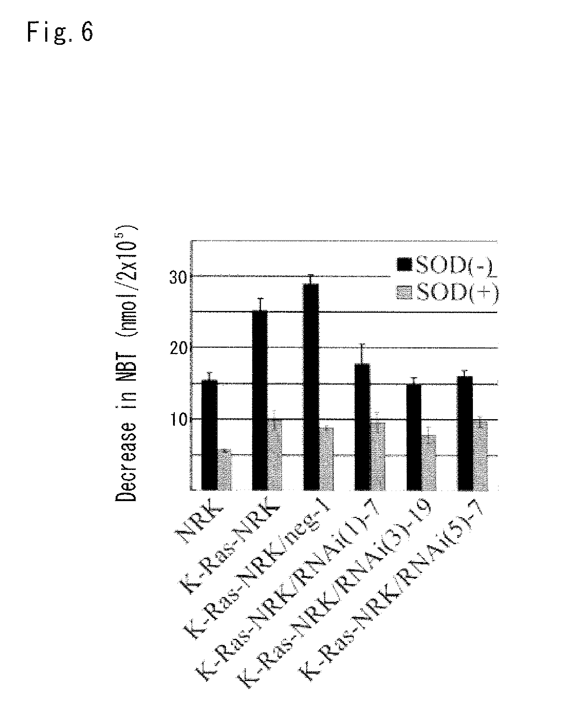

The present inventors measured superoxide production by NBT reduction analysis using the transformed cells of Example 4 in order to evaluate the role of Nox1 in superoxide production induced by mutant Ras. In the present example, the method of Suh et al. was used in the NBT (nitroblue tetrazolium) analysis (1). Namely, 0.2 ml of a Hanks solution containing 0.25% NBT was prepared with or without 40 units of superoxide dismutase (SOD), a reactive oxygen-digesting enzyme, in which 2x10⁵ cells were in turn suspended and treated at 37°C for 8 minutes. The treated cells were separated by low-speed centrifugation and solubilized by the addition of 0.5 ml of pyridine. Absorbance at 510 nm was measured to quantify a decrease in NBT (extinction coefficient of I 1,000/M/cm). The obtained data was shown in Fig. 6 with +/-s.e.m. from 3 measurements.

As a result of the analysis, the K-Ras-NRK and K-Ras-NRK/neg-1 increased NBT reduction, which was inhibited by the superoxide dismutase treatment, as compared with the NRK. By contrast, the K-Ras-NRK/RNAi(1)-7, K-Ras-NRK/RNAi(3)-19, and K-Ras-NRK/RNAi(5)-7 decreased the ROS production-stimulating effect of K-Ras oncogene to the level of the NRK cells (Fig. 6), thereby indicating that Nox1 is involved in an increase in superoxide production in mutant Ras-transformed cells.

### (Example 6)

### Confirmation of Nox1 expression decreased by RNAi(1), RNAi(3), and RNAi(5)

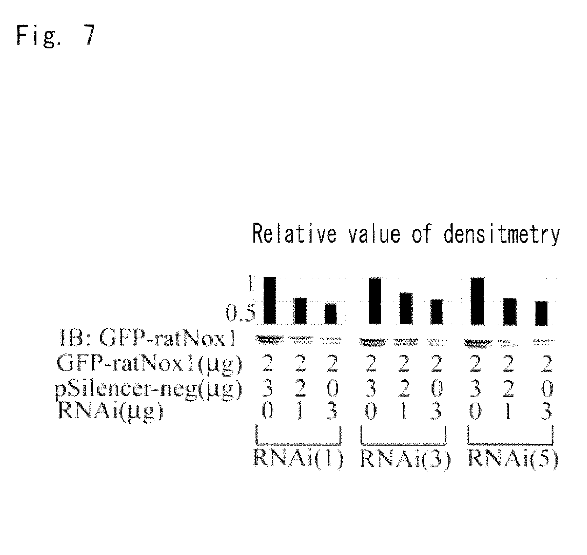

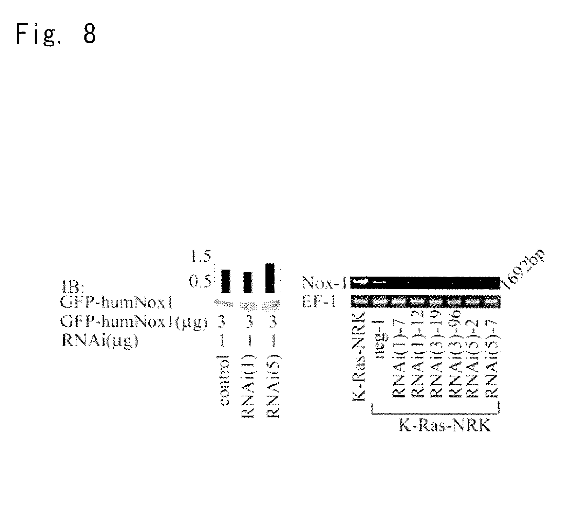

The present inventors conducted analysis in the following procedures in order to confirm Nox1 expression decreased by the RNAi(1), RNAi(3), and RNAi(5): GFP-rat Nox1 was subjected to cotransfection with each of the RNAi(1) to (5) and subsequently immunoblotting analysis was conducted in the same way as in Example 2 in order to evaluate the inhibitory effect of the RNAi(1), RNAi(3), and RNAi(5) on GFP-rat Nox1 expression. As shown in Fig. 7, it was revealed that an expected GFP-rat Nox1 fusion polypeptide is produced, and that the coexpression of the RNAi(1), RNAi (3), and RNAi(5) suppresses the production of a GFP-rat Nox1 fusion protein dependently on a dose of these vectors. GFP-Nox1 expression shown in Fig. 7 was quantitatively determined by immunoblotting using an anti-GFP antibody. Numerals in Fig. 7 denote the amount (µg) of DNA transfected. Both RNAi(1) and RNAi(5) are targeted for rat Nox1. The same approach as in Fig. 7 was used to show in the left panel of Fig. 8 that the RNAi(1) and RNAi(5) do not inhibit human Nox1, that is, the RNAi constructions have high specificity to their target gene.

Moreover, RT-PCR demonstrated that the mRNA expression of endogenous Nox1 is certainly inhibited in each of the K-Ras-NRK/RNAi(1)-7, K-Ras-NRK/RNAi(1)-12, K-Ras-NRK/RNAi(3)-19, K-Ras-NRK/RNAi(3)-96, K-Ras-NRK/RNAi(5)-2, K-Ras-NRK/RNAi(5)-7 cells, as compared to the K-Ras-NRK and K-Ras-NRK/neg-1 cells. The result is shown in the left panel of Fig. 8.

Sequences of PCR Primers used in Example 6
Forward Primer: 5'-ATGGGAAACTGGCTGGTTA-3' (SEQ ID NO: 26)
Reverse Primer: 5'-TCAGAACGTTTCTTTGTTGAA-3' (SEQ ID NO: 27)

The results of the present Example indicate that an increase in the expression rate of Nox1 gene and transformation with mutant Ras are associated with each other, as well as morphological changes caused by the constitutional changes of cytoskeletons and adhesive proteins.

### (Example 7)

### Influence of reactivation of Nox1 once inhibited by siRNA on cell

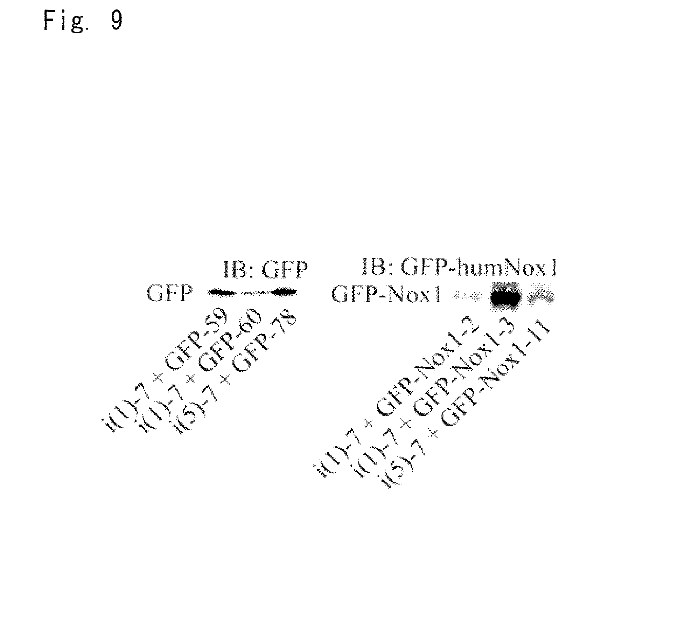

pEGFP-C (GFP) and pEGFP-human Nox1 (GFP-Nox1) were separately transfected into the K-Ras-NRKfRNAi(1)-7 and K-Ras-NRK/RNAi(5)-7 by the same approach as in Example 4, then immunoblotting was conducted by the same approach as in Example 6 (Fig. 9). As shown in Example 6 and the left panel of Fig. 8, neither RNAi(1) nor RNAi(5) inhibits human Nox1 expression. Therefore, even if the pEGFP-human Nox1 is transfected into the K-Ras-NRK/RNAi(1)-7 and K-Ras-NRK/RNAi(5)-7 cells, this human Nox1 is not inhibited by siRNA.

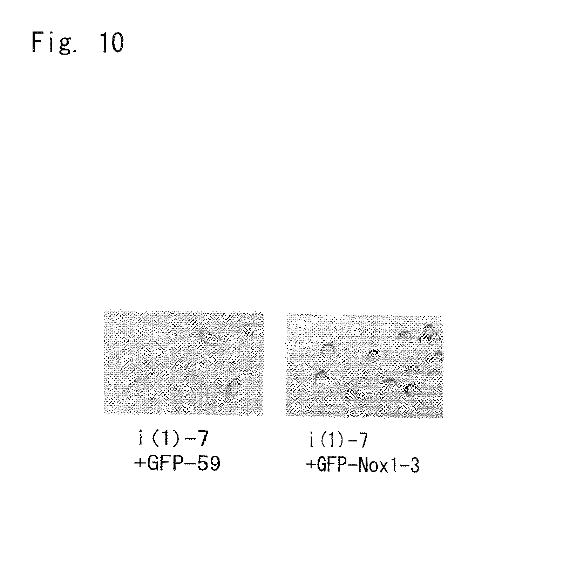

As shown in Fig. 10, the K-Ras-NRK/RNAi(1)-7 cells could not derepress siRNA and were oblate only by introducing the control vector therein (i(1)-7+GFP), whereas they morphologically returned to the spherical shape close to the original K-Ras-NRK cells by transfecting the pEGFP-human Nox1 therein (i(1)-7+GFP-Nox1-3) (Fig. 10).

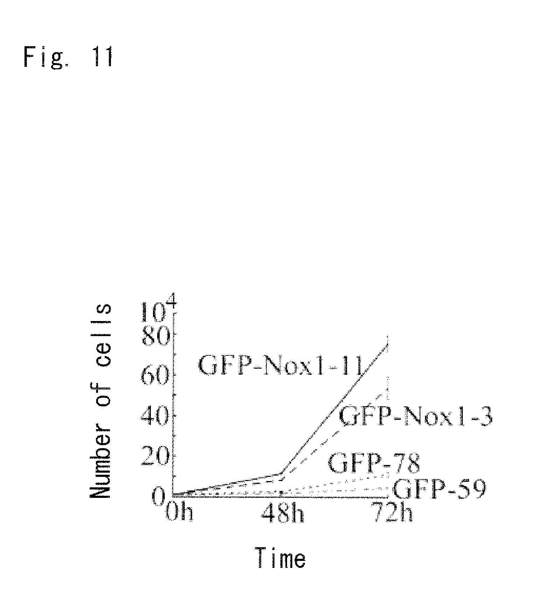

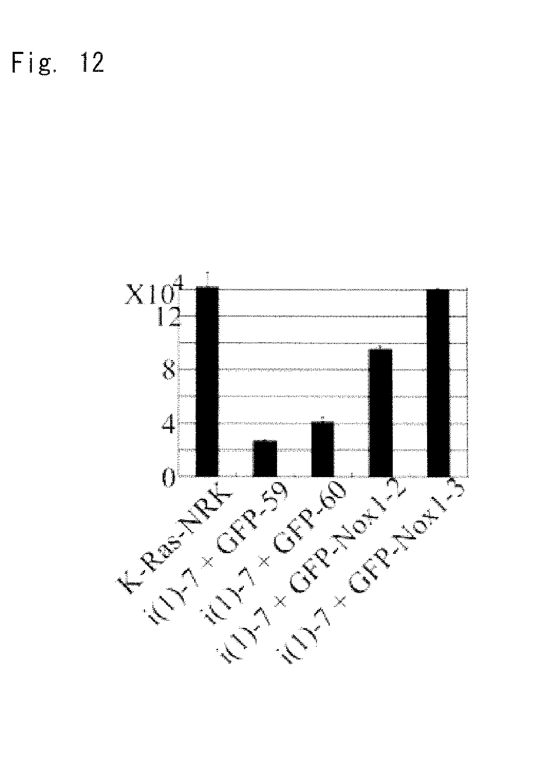

Growth curves obtained by the same approach as in Example 4 also exhibited accelerated growth in the pEGFP-human Nox1-incorporated K-Ras-NRK/RNAi(1)-7 (GFP-Nox1-3) and pEGFP-human Nox1-incorporated K-Ras-NRK/RNAi(5)-7 (GFF-Nox1-11), as compared with the control vector-incorporated K-Ras-NRK/RNAi(l)-7 (GFP-59) and the control vector-incorporated K-Ras-NRK/RNAi(5)-7 (GFP-78) (Fig. 11). Moreover, the soft agar culture assay in the same way as in Example 4 also demonstrated that colony formation in the pEGFP-human Nox1-incorporated K-Ras-NRK/RNAi(1)-7 (GFP-Nox1-2 and GFP-Nox1-3) recovered to the same level as that in the original K-Ras-NRK cells, as compared with the control vector-incorporated K-Ras-NRK/RNAi(1)-7 (i(1)-7+GFP-59 and I(1)-7+GFP-60) (Fig. 12).

### (Example 8)

### Influence of Nox1 gene expression on transformation with mutant Ras

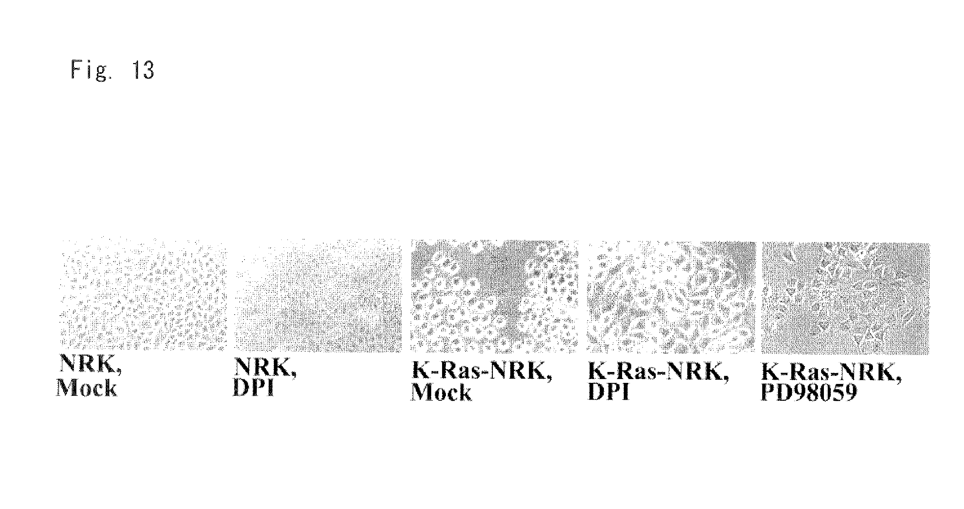

Diphenylene iodonium (DPI), a Nox1 inhibitor, was used to examine the influence of Nox1 gene expression on the morphologies of the cells transformed with mutant Ras. Namely, K-Ras-NRK cells and NRK cells were cultured overnight at 37°C in a medium containing 20 µM DPI or 30 µM PD98059 under a 5% CO₂-containing moisture environment to observe morphological changes. Photographs documenting the cell morphologies are shown in Fig. 13.

As shown in Fig. 13, the K-Ras-NRK cells transiently assumed a flat shape that was close to the shape of the NRK cells, when the K-Ras-NRK cells were treated overnight with flavoprotein inhibitor DPI or 10 mM antioxidant n-acetyl cysteine (data not shown). As further shown in Fig. 13, such morphological changes were also observed in the K-Ras-NRK cells treated with the PD98059. Thus, these results indicated that an increase in Nox1 gene expression is essential to Ras-MAPKK-MAPK pathway-promoted transformation with mutant Ras.

### (Example 9)

### Influence of siRNA on tumor formation caused by mutant Ras

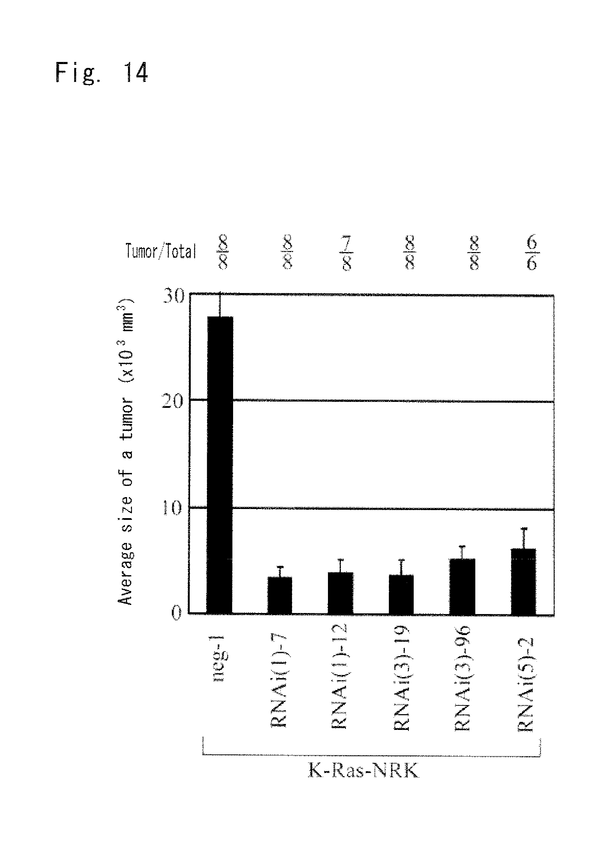

The K-Ras-NRK/neg-1, K-Ras-NRK/RNAi(1)-7, K-Ras-NRK/RNAi(1)-12, K-Ras-NRK/RNAi(3)-19, K-Ras-NRK/RNAi(3)-96, and K-Ras-NRK/RNAi(5)-2 cells were separately transplanted into athymic mice to observe tumor formation. Namely, 10⁶ cells each of the cells were suspended in 0.2 ml PBS and then subcutaneously transplanted into the nude mice (athymic: Nu/Nu). Then, tumor formation in all of the nude mice was observed over 1 month, while the volume of the formed tumor was measured. The result was shown in Fig. 14. In Fig. 14, the solidly shaded bars denote the volume of tumor; the error bars denote s.e.m.; and the fractions denote the proportion of tumor-bearing mice to all the mice used (tumor/total).

As shown in Fig. 14, the K-Ras-NRK/neg-1 formed active tumor within 2 weeks. By contrast, the K-Ras-NRKlRNAi(1)-7, K-Ras-NRKlRNAi(1)-12, K-Ras-NRK/RNAi(3)-19, and K-Ras-NRK/RNAi(3)-96 remarkably suppressed tumor growth. When histologically observed by anatomy, the tumor formed in the K-Ras-NRK/neg-1 resulted in increased blood vessels. This is consistent with the possibility that Nox1 gene induces angiogenesis caused by increase in vascular endothelial growth factor production (1). The small tumor formed in the K-Ras-NRK/RNAi(1)-7, K-Ras-NRK/RNAi(1)-12, K-Ras-NRK/RNAi(3)-19, and K-Ras-NRK/RNAi(3)-96 exhibited signs of necrosis in most of the cells (data not shown).

As seen from these experimental results, mutant Ras (oncogene) elevates mitogenic oxidase Nox1 via MAPKK-MAPK pathways, whereas Nox1 gene expression is essential to transformation of cells, cancer formation, and its progression caused by the Ras. Namely, the studies conducted by the present inventors revealed a molecular mechanism in which a Nox1 polypeptide plays an essential role as a redox signal molecule in transformation with mutant Ras.

Nox family proteins Nox1 to 5, Gp91phox homologues, were identified from non-phagocytic cells and reported to respectively have specific functions in various cell processes (1 and 5 to 9). Among the Nox family, the Nox1 gene is unique in that it codes for transmembrane oxidase that mediates the mitogenic signals from growth factors (1 and 4) and oncogenes.

Considering the involvement of ROS in the promotion of tumor growth and the possibility of ROS production for inducing ROS-producing enzymes in malignant cancer cells (7), Nox1 gene and its polypeptides are probably capable of suppressing cancer progression and serving as target molecules that allow the cancer therapy.

The SEQ ID NOs in the sequence listing of the present specification represent the following sequences:
SEQ ID NO: 1 represents the nucleotide sequence of mRNA transcribed from a human Nox1 gene or cDNA;
SEQ ID NO: 2 represents the amino acid sequence of a polypeptide coded by the human Nox1 gene;
SEQ ID NO: 3 represents the nucleotide sequence of mRNA transcribed from a rat Nox1 gene or cDNA;
SEQ ID NO: 4 represents the amino acid sequence of a polypeptide coded by the rat Nox1 gene;
SEQ ID NO: 5 represents the nucleotide sequence of a forward primer corresponding to a Nox1 gene used in a diagnostic method for human cancer;
SEQ ID NO: 6 represents the nucleotide sequence of a reverse primer corresponding to a Nox1 gene used in a diagnostic method for human cancer;
SEQ ID NO: 7 represents the sequence of a forward primer used in the detection of a human Nox1 gene by real-time quantitative polymerase chain reaction;
SEQ ID NO: 8 represents the sequence of a reverse primer used in the detection of a human Nox1 gene by real-time quantitative polymerase chain reaction;
SEQ ID NO: 9 represents the sequence of a TaqMan probe used in the detection of a human Nox1 gene by real-time quantitative polymerase chain reaction;
SEQ ID NO: 10 represents the nucleotide sequence of siRNA for a human Nox1 gene of the present invention;
SEQ ID NO: 11 represents the nucleotide sequence of siRNA for a human Nox1 gene of the present invention;
SEQ ID NO: 12 represents the nucleotide sequence of siRNA for a rat Nox1 gene of the present invention;
SEQ ID NO: 13 represents the nucleotide sequence of siRNA for a rat Nox1 gene of the present invention;
SEQ ID NO: 14 represents the nucleotide sequence of siRNA for a rat Nox1 gene of the present invention;
SEQ ID NO: 15 represents the nucleotide sequence of a forward primer used in Example 1 for detecting the presence or absence of Nox1 expression by real-time quantitative polymerase chain reaction;
SEQ ID NO: 16 represents the nucleotide sequence of a reverse primer used in Example 1 for detecting the presence or absence of Nox1 expression by real-time quantitative polymerase chain reaction;
SEQ ID NO: 17 represents the nucleotide sequence of a TaqMan MGB probe used in Example 1 for detecting the presence or absence of Nox1 expression by real-time quantitative polymerase chain reaction;
SEQ ID NO: 18 represents the nucleotide sequence of an oligonucleotide that constitutes an siRNA construction used in Example 4 for examining the involvement of a Nox1 gene in transformation with a mutant Ras gene;
SEQ TD NO: 19 represents the nucleotide sequence of an oligonucleotide that constitutes an siRNA construction used in Example 4 for examining the involvement of a Nox1 gene in transformation with a mutant Ras gene;
SEQ ID NO: 20 represents the nucleotide sequence of an oligonucleotide that constitutes an siRNA construction used in Example 4 for examining the involvement of a Nox1 gene in transformation with a mutant Ras gene;
SEQ ID NO: 21 represents the nucleotide sequence of an oligonucleotide that constitutes an siRNA construction used in Example 4 for examining the involvement of a Nox1 gene in transformation with a mutant Ras gene;
SEQ ID NO: 22 represents the nucleotide sequence of an oligonucleotide that constitutes an siRNA construction used in Example 4 for examining the involvement of a Nox1 gene in transformation with a mutant Ras gene;
SEQ ID NO: 23 represents the nucleotide sequence of an oligonucleotide that constitutes an siRNA construction used in Example 4 for examining the involvement of a Nox1 gene in transformation with a mutant Ras gene;
SEQ ID NO: 24 represents the nucleotide sequence of an M13 primer used in Example 4 for confirming the transfection of the siRNA constructions for a Nox1 gene;
SEQ ID NO: 25 represents the nucleotide sequence of a 3.0 Rev primer used in Example 4 for confirming the transfection of the siRNA constructions for a Nox1 gene;
SEQ ID NO: 26 represents the nucleotide sequence of a forward primer used in Example 6 for confirming a decrease in Nox1 expression; and
SEQ ID NO: 27 represents the nucleotide sequence of a reverse primer used in Example 6 for confirming a decrease in Nox1 expression.

### (References)

1. Suh, Y-A, et al. Nature 401,79-82 (1999)
2. Arnold, R. S. et al. Proc. Natl. Acad. Sci. USA. 98, 5550-5555 (2001)
3. Arbiser, J. L. et al. Proc. Natl. Acad. Sci. USA. 99, 715-720 (2001)
4. Lassegue B. et al. Circ. Res. 88, 888-894 (2001)
5. Lambeth, J. D., Dheng, G., Arnold, R. S. & Edens, W. A. TIBS. 25, 459-461 (2000)
6. Royer-Pokora, B. et al. Nature 322, 32-38 (1986)
7. Kikuchi, H., Hikage, M., Miyashita, H. & Fulumoto, M. Gene 269,131-140 (2001)

## Claims

1. A composition for producing an antibody, comprising: (1) a polypeptide comprising the amino acid sequence of SEQ ID NO: 2; (2) a polypeptide having an amino acid sequence mutated from the amino acid sequence of SEQ ID NO: 2 by the substitution, deletion, addition, and/or insertion of one or more amino acid residues and inducing the production of an antibody specific to the polypeptide comprising the amino acid sequence of SEQ ID NO: 2: or (3) a polypeptide fragment having a partial sequence of the polypeptide of (1) or (2) and inducing the production of an antibody specific to the polypeptide comprising the amino acid sequence of SEQ ID NO: 2.

2. The composition according to claim 1. wherein the antibody is an antibody for detecting a human cancer cell.

3. The composition according to claim 1, comprising a polypeptide comprising the amino acid sequence of SEQ ID NO: 2.

4. The composition according to claim 1. comprising a polypeptide having a partial sequence of the amino acid sequence of SEQ ID NO: 2 and inducing the production of an antibody specific to the polypeptide comprising the amino acid sequence of SEQ ID NO: 2.

5. The composition according to claim 1, comprising a polypeptide fragment having a partial sequence of the amino acid sequence of SEQ ID NO: 2 and inducing the production of an antibody specific to the polypeptide comprising the amino acid sequence of SEQ ID NO: 2.

6. A method for producing an antibody specific to a polypeptide comprising the amino acid sequence of SEQ ID NO: 2, comprising administering a composition according to claim 1 to a mammal.

7. An antibody specific to a polypeptide comprising the amino acid sequence of SEQ ID NO: 2.

8. The antibody according to claim 7, wherein the antibody is a human/mouse chimeric antibody, a humanized antibody, or a human antibody.

9. The antibody according to claim 7, wherein the antibody is a polyclonal antibody or a monoclonal antibody.

10. A diagnostic method for cancer, comprising bringing an antibody according to claim 7 into contact with a biological sample.

11. A diagnostic kit for cancer, comprising an antibody according to claim 7.

12. A pharmaceutical composition for cancer therapy, comprising an antibody according to claim 7.

13. The pharmaceutical composition for cancer therapy according to claim 12, wherein the antibody is a human/mouse chimeric antibody, a humanized antibody, or a human antibody.

14. The pharmaceutical composition for cancer therapy according to claim 12, wherein the antibody is a polyclonal antibody or a monoclonal antibody.

15. The pharmaceutical composition for cancer therapy according to claim 12, further comprising an appropriate carrier.

16. A diagnostic method for cancer, **characterized by** detecting a polynucleotide comprising the nucleotide sequence of SFQ ID NO: 1 or a fragment thereof.

17. The diagnostic method for cancer according to claim 16, wherein the polynucleotide or the fragment thereof is detected by polymerase chain reaction (PCR) or real-time quantitative polymerase chain reaction.

18. The diagnostic method for cancer according to claim 17, wherein the detection is performed by PCR using a sense strand fragment corresponding to the nucleotide sequence of SEQ ID NO: 1 as a forward primer and an antisense strand fragment corresponding to the nucleotide sequence of SEQ ID NO: 1 as a reverse primer.

19. The diagnostic method for cancer according to claim 18. wherein the forward primer has 14 to 60 bases in length and the reverse primer has 14 to 60 bases in length.

20. The diagnostic method for cancer according to claim 18, wherein the diagnostic method uses the following forward and reverse primers:
the forward primer of 5'- GGAGCAGGAATTGGGGTCAC -3' (SEQ ID NO: 5); and
the reverse primer of 5'- TTGCTGTCCCATCCGGTGAG -3' (SEQ ID NO: 6).

21. The diagnostic method for cancer according to claim 17, wherein the detection is performed by real-time quantitative polymerase chain reaction using a sense strand fragment corresponding to the nucleotide sequence of SEQ ID NO: 1 as a forward primer and an antisense strand fragment corresponding to the nucleotide sequence of SEQ ID NO: 1 as a reverse primer.

22. The diagnostic method for cancer according to claim 21, wherein the diagnostic method uses the following forward and reverse primers and TaqMan probe:
the forward primer of 5'- CCACTGTAGGCGCCCTAAGTT -3' (SEQ ID NO: 7);
the reverse primer of 5'- AAGAATGACCGGTGCAAGGA -3' (SEQ ID NO: 8); and
the TaqMan probe of 5'- AAGGGCATCCCCCTGAGTCTTGGAA -3' (SEQ ID NO: 9).

23. siRNA corresponding to a polynucleotide comprising the nucleotide sequence of SEQ ID NO: 1 or a fragment thereof.

24. The siRNA according to claim 23, corresponding to a polynucleotide comprising a nucleotide sequence at positions from 71 to 1615 of SEQ ID NO: 1 or a fragment thereof.

25. The siRNA according to claim 23, wherein the siRNA has a nucleotide sequence from 8 to 30 bp in length.

26. The siRNA according to claim 23, wherein the siRNA consists of a nucleotide sequence selected from the group consisting of the following nucleotide sequences of SEQ ID NOs: 10 to 14:

5'-GCGTGGCTTCAGCATGGAATTCAAGAGATTCCATGCTGAAGCCACGCTTTTT
TGGAAA-3' (SEQ ID NO: 10);

5'-GGGCTTTCGAACAACAATATTCAAGAGATATTGTTGTTCGAAAGCCCTTTTT
TGGAAA-3' (SEQ ID NO: 11);

5'-GTTATGAGAAGTCTGACAAGTTCAAGAGACTTGTCAGACTTCTCATAATTTT
TTGGAAA-3' (SEQ ID NO: 12);

5'-GATTCTTGGCTAAATCCCATTCAAGAGATGGGATTTAGCCAAGAATCTTTTT
TGGAAA-3' (SEQ ID NO: 13); and

5'-GGACATTTGAACAACAGCATTCAAGAGATGCTGTTGTTCAAATGTCCTTTTT
TGGAAA-3' (SEQ ID NO: 14).

27. A pharmaceutical composition for cancer therapy, comprising: siRNA corresponding to a polynucleotide comprising the nucleotide sequence of SEQ ID NO: 1 or a fragment thereof; and siRNA corresponding to a polynucleotide comprising a nucleotide sequence at positions from 71 to 1615 ofSEQ ID NO: 1 or a fragment thereof.

28. A pharmaceutical composition for cancer therapy, comprising a cell transformed with siRNA corresponding to a polynucleotide comprising the nucleotide sequence of SEQ ID NO: 1 or a fragment thereof; and siRNA corresponding to a polynucleotide comprising a nucleotide sequence at positions from 71 to 1615 of SEQ ID NO: 1 or a fragment thereof.

29. The pharmaceutical composition for cancer therapy according to claim 28, wherein the cell to be transformed is a cell taken out of a patient to be treated.

30. A method for producing a cell for cancer therapy, comprising preparing a human cell and transforming the cell with siRNA corresponding to a polynucleotide comprising the nucleotide sequence of SEQ ID NO: 1 or a fragment thereof; and siRNA corresponding to a polynucleotide comprising a nucleotide sequence at positions from 71 to 1615 of SEQ ID NO: 1 or a fragment thereof.

31. The method for producing a cell for cancer therapy according to claim 30, wherein the human cell is a cell taken out of a patient to be treated.

32. A screening method for a cancer cell growth inhibitor targeted for a Nox1 gene, comprising: transfecting a cell having a mutant Ras gene with a Nox1 gene; bringing the transformed cell into contact with a substance to be screened; and detecting the expression of the Nox1 gene and the inactivation of Nox1 activity.

33. The screening method according to claim 32, comprising culturing the transformed cell together with the substance to be screened.

34. The screening method according to claim 32, wherein the expression of the Nox1 gene is detected by detecting mRNA by real-time quantitative polymerase chain reaction or detecting a polypeptide or a peptide fragment thereof coded by the Nox 1 gene with an antibody.

35. The screening method according to claim 32, wherein the expression of the Nox1 gene is detected by observing morphological changes in the transformed cell.

36. The screening method according to claim 32, wherein the cell having a mutant Ras gene is an H-Ras-NIH3T3 cell or a K-Ras-NRK cell.

37. The screening method according to claim 32, wherein the transfection of the Nox1 gene is performed using pEGFP-C1 (K-Ras-NRK/GFP) or pEGFP-C1-Nox1 (K-Ras-NRK/GFP-Nox1).

38. A cell having a mutant Ras gene, which is transfected with a Nox1 gene.


SEQUENCE LISTING

<110> Kureha Corporation
KAMATA, Toru
MITSUSHITA, Junji

<120> Antibodies to Nox1 polypeptide, method for the detection of cancer using
Nox1 gene and method for screening substances suppressing cancer growth

<130> HMJ04186EP

<140> EP 04748306.0
<141> 2004-08-06

<150> JP 2003-308658
<151> 2003-09-01

<160> 27

<170> PatentIn version 3.1

<210> 1
<211> 1734
<212> DNA
<213> Homo sapiens

<220>
<221> CDS
<222> (71)..(1618)
<223> Human Nox1 polypeptide of SEQ NO:2

<400> 1
ggacctctcc agaatccgga ttgctgaatc ttccctgttg cctagaaggg ctccaaacca 60

cctcttgaca atg gga aac tgg gtg gtt aac cac tgg ttt tca gtt ttg 109
Met Gly Asn Trp Val Val Asn His Trp Phe Ser Val Leu
1               5                   10

ttt ctg gtt gtt tgg tta ggg ctg aat gtt ttc ctg ttt gtg gat gcc 157
Phe Leu Val Val Trp Leu Gly Leu Asn Val Phe Leu Phe Val Asp Ala
    15                  20                  25

ttc ctg aaa tat gag aag gcc gac aaa tac tac tac aca aga aaa atc 205
Phe Leu Lys Tyr Glu Lys Ala Asp Lys Tyr Tyr Tyr Thr Arg Lys Ile
30                  35                  40                  45

ctt ggg tca aca ttg gcc tgt gcc cga gcg tct gct ctc tgc ttg aat 253
Leu Gly Ser Thr Leu Ala Cys Ala Arg Ala Ser Ala Leu Cys Leu Asn
                50                  55                  60

ttt aac agc acg ctg atc ctg ctt cct gtg tgt cgc aat ctg ctg tcc 301
Phe Asn Ser Thr Leu Ile Leu Leu Pro Val Cys Arg Asn Leu Leu Ser
            65                  70                  75

ttc ctg agg ggc acc tgc tca ttt tgc agc cgc aca ctg aga aag caa 349
Phe Leu Arg Gly Thr Cys Ser Phe Cys Ser Arg Thr Leu Arg Lys Gln
        80                  85                  90

ttg gat cac aac ctc acc ttc cac aag ctg gtg gcc tat atg atc tgc 397
Leu Asp His Asn Leu Thr Phe His Lys Leu Val Ala Tyr Met Ile Cys

95                 100                 105

cta cat aca gct att cac atc att gca cac ctg ttt aac ttt gac tgc       445
Leu His Thr Ala Ile His Ile Ile Ala His Leu Phe Asn Phe Asp Cys
110                 115                 120                 125

tat agc aga agc cga cag gcc aca gat ggc tcc ctt gcc tcc att ctc       493
Tyr Ser Arg Ser Arg Gln Ala Thr Asp Gly Ser Leu Ala Ser Ile Leu
                130                 135                 140

tcc agc cta tct cat gat gag aaa aag ggg ggt tct tgg cta aat ccc       541
Ser Ser Leu Ser His Asp Glu Lys Lys Gly Gly Ser Trp Leu Asn Pro
            145                 150                 155

atc cag tcc cga aac acg aca gtg gag tat gtg aca ttc acc agc att       589
Ile Gln Ser Arg Asn Thr Thr Val Glu Tyr Val Thr Phe Thr Ser Ile
        160                 165                 170

gct ggt ctc act gga gtg atc atg aca ata gcc ttg att ctc atg gta       637
Ala Gly Leu Thr Gly Val Ile Met Thr Ile Ala Leu Ile Leu Met Val
    175                 180                 185

act tca gct act gag ttc atc cgg agg agt tat ttt gaa gtc ttc tgg       685
Thr Ser Ala Thr Glu Phe Ile Arg Arg Ser Tyr Phe Glu Val Phe Trp
190                 195                 200                 205

tat act cac cac ctt ttt atc ttc tat atc ctt ggc tta ggg att cac       733
Tyr Thr His His Leu Phe Ile Phe Tyr Ile Leu Gly Leu Gly Ile His
                210                 215                 220

ggc att ggt gga att gtc cgg ggt caa aca gag gag agc atg aat gag       781
Gly Ile Gly Gly Ile Val Arg Gly Gln Thr Glu Glu Ser Met Asn Glu
            225                 230                 235

agt cat cct cgc aag tgt gca gag tct ttt gag atg tgg gat gat cgt       829
Ser His Pro Arg Lys Cys Ala Glu Ser Phe Glu Met Trp Asp Asp Arg
        240                 245                 250

gac tcc cac tgt agg cgc cct aag ttt gaa ggg cat ccc cct gag tct       877
Asp Ser His Cys Arg Arg Pro Lys Phe Glu Gly His Pro Pro Glu Ser
    255                 260                 265

tgg aag tgg atc ctt gca ccg gtc att ctt tat atc tgt gaa agg atc       925
Trp Lys Trp Ile Leu Ala Pro Val Ile Leu Tyr Ile Cys Glu Arg Ile
270                 275                 280                 285

ctc cgg ttt tac cgc tcc cag cag aag gtt gtg att acc aag gtt gtt       973
Leu Arg Phe Tyr Arg Ser Gln Gln Lys Val Val Ile Thr Lys Val Val
                290                 295                 300

atg cac cca tcc aaa gtt ttg gaa ttg cag atg aac aag cgt ggc ttc      1021
Met His Pro Ser Lys Val Leu Glu Leu Gln Met Asn Lys Arg Gly Phe
            305                 310                 315

agc atg gaa gtg ggg cag tat atc ttt gtt aat tgc ccc tca atc tct      1069
Ser Met Glu Val Gly Gln Tyr Ile Phe Val Asn Cys Pro Ser Ile Ser
        320                 325                 330

ctc ctg gaa tgg cat cct ttt act ttg acc tct gct cca gag gaa gat      1117
Leu Leu Glu Trp His Pro Phe Thr Leu Thr Ser Ala Pro Glu Glu Asp
    335                 340                 345

ttc ttc tcc att cat atc cga gca gca ggg gac tgg aca gaa aat ctc     1165
Phe Phe Ser Ile His Ile Arg Ala Ala Gly Asp Trp Thr Glu Asn Leu
350                 355                 360                 365

ata agg gct ttc gaa caa caa tat tca cca att ccc agg att gaa gtg     1213
Ile Arg Ala Phe Glu Gln Gln Tyr Ser Pro Ile Pro Arg Ile Glu Val
                370                 375                 380

gat ggt ccc ttt ggc aca gcc agt gag gat gtt ttc cag tat gaa gtg     1261
Asp Gly Pro Phe Gly Thr Ala Ser Glu Asp Val Phe Gln Tyr Glu Val
            385                 390                 395

gct gtg ctg gtt gga gca gga att ggg gtc acc ccc ttt gct tct atc     1309
Ala Val Leu Val Gly Ala Gly Ile Gly Val Thr Pro Phe Ala Ser Ile
        400                 405                 410

ttg aaa tcc atc tgg tac aaa ttc cag tgt gca gac cac aac ctc aaa     1357
Leu Lys Ser Ile Trp Tyr Lys Phe Gln Cys Ala Asp His Asn Leu Lys
    415                 420                 425

aca aaa aag gtt ggt cat gca gca tta aac ttt gac aag gcc act gac     1405
Thr Lys Lys Val Gly His Ala Ala Leu Asn Phe Asp Lys Ala Thr Asp
430                 435                 440                 445

atc gtg aca ggt ctg aaa cag aaa acc tcc ttt ggg aga cca atg tgg     1453
Ile Val Thr Gly Leu Lys Gln Lys Thr Ser Phe Gly Arg Pro Met Trp
                450                 455                 460

gac aat gag ttt tct aca ata gct acc tcc cac ccc aag tct gta gtg     1501
Asp Asn Glu Phe Ser Thr Ile Ala Thr Ser His Pro Lys Ser Val Val
            465                 470                 475

gga gtt ttc tta tgt ggc cct cgg act ttg gca aag agc ctg cgc aaa     1549
Gly Val Phe Leu Cys Gly Pro Arg Thr Leu Ala Lys Ser Leu Arg Lys
        480                 485                 490

tgc tgt cac cga tat tcc agt ctg gat cct aga aag gtt caa ttc tac     1597
Cys Cys His Arg Tyr Ser Ser Leu Asp Pro Arg Lys Val Gln Phe Tyr
    495                 500                 505

ttc aac aaa gaa aat ttt tga gttataggaa taaggacggt aatctgcatt     1648
Phe Asn Lys Glu Asn Phe
510                 515

ttgtctcttt gtatcttcag taatttactt ggtctcgtca ggtttgagca gtcactttag     1708

gataagaatg tgcctctcaa gccttg                                          1734


<210>  2
<211>  515
<212>  PRT
<213>  Homo sapiens

<400>  2

Met Gly Asn Trp Val Val Asn His Trp Phe Ser Val Leu Phe Leu Val
1               5                   10                  15

Val Trp Leu Gly Leu Asn Val Phe Leu Phe Val Asp Ala Phe Leu Lys
            20                  25                  30

Tyr Glu Lys Ala Asp Lys Tyr Tyr Tyr Thr Arg Lys Ile Leu Gly Ser
        35                  40                  45

Thr Leu Ala Cys Ala Arg Ala Ser Ala Leu Cys Leu Asn Phe Asn Ser
    50                  55                  60

Thr Leu Ile Leu Leu Pro Val Cys Arg Asn Leu Leu Ser Phe Leu Arg
65                  70                  75                  80

Gly Thr Cys Ser Phe Cys Ser Arg Thr Leu Arg Lys Gln Leu Asp His
                85                  90                  95

Asn Leu Thr Phe His Lys Leu Val Ala Tyr Met Ile Cys Leu His Thr
            100                 105                 110

Ala Ile His Ile Ile Ala His Leu Phe Asn Phe Asp Cys Tyr Ser Arg
        115                 120                 125

Ser Arg Gln Ala Thr Asp Gly Ser Leu Ala Ser Ile Leu Ser Ser Leu
    130                 135                 140

Ser His Asp Glu Lys Lys Gly Gly Ser Trp Leu Asn Pro Ile Gln Ser
145                 150                 155                 160

Arg Asn Thr Thr Val Glu Tyr Val Thr Phe Thr Ser Ile Ala Gly Leu
                165                 170                 175

Thr Gly Val Ile Met Thr Ile Ala Leu Ile Leu Met Val Thr Ser Ala
            180                 185                 190

Thr Glu Phe Ile Arg Arg Ser Tyr Phe Glu Val Phe Trp Tyr Thr His
        195                 200                 205

His Leu Phe Ile Phe Tyr Ile Leu Gly Leu Gly Ile His Gly Ile Gly
    210                 215                 220

Gly Ile Val Arg Gly Gln Thr Glu Glu Ser Met Asn Glu Ser His Pro
225                 230                 235                 240

Arg Lys Cys Ala Glu Ser Phe Glu Met Trp Asp Asp Arg Asp Ser His
                245                 250                 255

Cys Arg Arg Pro Lys Phe Glu Gly His Pro Pro Glu Ser Trp Lys Trp

260                 265                 270

Ile Leu Ala Pro Val Ile Leu Tyr Ile Cys Glu Arg Ile Leu Arg Phe
        275                 280                 285

Tyr Arg Ser Gln Gln Lys Val Val Ile Thr Lys Val Val Met His Pro
    290                 295                 300

Ser Lys Val Leu Glu Leu Gln Met Asn Lys Arg Gly Phe Ser Met Glu
305                 310                 315                 320

Val Gly Gln Tyr Ile Phe Val Asn Cys Pro Ser Ile Ser Leu Leu Glu
                325                 330                 335

Trp His Pro Phe Thr Leu Thr Ser Ala Pro Glu Glu Asp Phe Phe Ser
            340                 345                 350

Ile His Ile Arg Ala Ala Gly Asp Trp Thr Glu Asn Leu Ile Arg Ala
        355                 360                 365

Phe Glu Gln Gln Tyr Ser Pro Ile Pro Arg Ile Glu Val Asp Gly Pro
    370                 375                 380

Phe Gly Thr Ala Ser Glu Asp Val Phe Gln Tyr Glu Val Ala Val Leu
385                 390                 395                 400

Val Gly Ala Gly Ile Gly Val Thr Pro Phe Ala Ser Ile Leu Lys Ser
                405                 410                 415

Ile Trp Tyr Lys Phe Gln Cys Ala Asp His Asn Leu Lys Thr Lys Lys
            420                 425                 430

Val Gly His Ala Ala Leu Asn Phe Asp Lys Ala Thr Asp Ile Val Thr
        435                 440                 445

Gly Leu Lys Gln Lys Thr Ser Phe Gly Arg Pro Met Trp Asp Asn Glu
    450                 455                 460

Phe Ser Thr Ile Ala Thr Ser His Pro Lys Ser Val Val Gly Val Phe
465                 470                 475                 480

Leu Cys Gly Pro Arg Thr Leu Ala Lys Ser Leu Arg Lys Cys Cys His
                485                 490                 495

Arg Tyr Ser Ser Leu Asp Pro Arg Lys Val Gln Phe Tyr Phe Asn Lys
            500                 505                 510

Glu Asn Phe
        515

<210>  3
<211>  2577
<212>  DNA
<213>  Rattus norvegicus

<220>
<221>  CDS
<222>  (128)..(1819)
<223>  Rat Nox1 polypeptide of SEQ NO:4

<400>  3
ttctgagtag gtgtgcattt gagtgtcata aagacatata tcttgagcta gacagaagtt      60

cctatcctga aggatcccat cagagaaacc agattgctcc taagaggctc cagacctcca     120

tttgaca atg gga aac tgg ctg gtt aac cac tgg ctc tca gtt ttg ttt      169
        Met Gly Asn Trp Leu Val Asn His Trp Leu Ser Val Leu Phe
        1               5                   10

ctg gtt tct tgg ttg ggg ctg aac att ttt ctg ttt gtg tac gtc ttc      217
Leu Val Ser Trp Leu Gly Leu Asn Ile Phe Leu Phe Val Tyr Val Phe
15                  20                  25                  30

ctg aat tat gag aag tct gac aag tac tat tac acg aga gaa att ctc      265
Leu Asn Tyr Glu Lys Ser Asp Lys Tyr Tyr Tyr Thr Arg Glu Ile Leu
                35                  40                  45

gga act gcc ttg gcc ttg gcc aga gca tct gct ttg tgc ctg aat ttt      313
Gly Thr Ala Leu Ala Leu Ala Arg Ala Ser Ala Leu Cys Leu Asn Phe
            50                  55                  60

aac agc atg gtg atc ctg att cct gtg tgt cga aat ctg ctc tcc ttc      361
Asn Ser Met Val Ile Leu Ile Pro Val Cys Arg Asn Leu Leu Ser Phe
        65                  70                  75

ctg agg ggc acc tgc tca ttt tgc aac cac acg ctg aga aag cca ttg      409
Leu Arg Gly Thr Cys Ser Phe Cys Asn His Thr Leu Arg Lys Pro Leu
    80                  85                  90

gat cac aac ctc acc ttc cat aag ctg gtg gca tat atg atc tgc ata      457
Asp His Asn Leu Thr Phe His Lys Leu Val Ala Tyr Met Ile Cys Ile
95                  100                 105                 110

ttc aca gct att cat atc att gca cat cta ttt aac ttt gaa cgc tac      505
Phe Thr Ala Ile His Ile Ile Ala His Leu Phe Asn Phe Glu Arg Tyr
                115                 120                 125

agt aga agc caa cag gcc atg gat gga tct ctt gcc tct gtt ctc tcc      553
Ser Arg Ser Gln Gln Ala Met Asp Gly Ser Leu Ala Ser Val Leu Ser
            130                 135                 140

agc cta ttc cat ccc gag aaa gaa gat tct tgg cta aat ccc atc cag      601
Ser Leu Phe His Pro Glu Lys Glu Asp Ser Trp Leu Asn Pro Ile Gln
        145                 150                 155

tct cca aac gtg aca gtg atg tat gca gca ttt acc agt att gct ggc 649
Ser Pro Asn Val Thr Val Met Tyr Ala Ala Phe Thr Ser Ile Ala Gly
    160                 165                 170

ctt act gga gtg gtc gcc act gtg gct ttg gtt ctc atg gta act tca 697
Leu Thr Gly Val Val Ala Thr Val Ala Leu Val Leu Met Val Thr Ser
175                 180                 185                 190

gct atg gag ttt atc cgc agg aat tat ttt gag ctc ttc tgg tat aca 745
Ala Met Glu Phe Ile Arg Arg Asn Tyr Phe Glu Leu Phe Trp Tyr Thr
                195                 200                 205

cat cac ctt ttc atc atc tat atc atc tgc tta ggg atc cat ggc ctg 793
His His Leu Phe Ile Ile Tyr Ile Ile Cys Leu Gly Ile His Gly Leu
            210                 215                 220

ggg ggg att gtc cgg ggt caa aca gaa gag agc atg agt gaa agt cat 841
Gly Gly Ile Val Arg Gly Gln Thr Glu Glu Ser Met Ser Glu Ser His
        225                 230                 235

ccc cgc aac tgt tca tac tct ttc cac gag tgg gat aag tat gaa agg 889
Pro Arg Asn Cys Ser Tyr Ser Phe His Glu Trp Asp Lys Tyr Glu Arg
    240                 245                 250

agt tgc agg agt cct cat ttt gtg ggg caa ccc cct gag tct tgg aag 937
Ser Cys Arg Ser Pro His Phe Val Gly Gln Pro Pro Glu Ser Trp Lys
255                 260                 265                 270

tgg atc ctc gcg ccg att gct ttt tat atc ttt gaa agg atc ctt cgc 985
Trp Ile Leu Ala Pro Ile Ala Phe Tyr Ile Phe Glu Arg Ile Leu Arg
                275                 280                 285

ttt tat cgc tcc cgg cag aag gtc gtg att acc aag gtt gtc atg cac 1033
Phe Tyr Arg Ser Arg Gln Lys Val Val Ile Thr Lys Val Val Met His
            290                 295                 300

cca tgt aaa gtt ttg gaa ttg cag atg agg aag cgg ggc ttt act atg 1081
Pro Cys Lys Val Leu Glu Leu Gln Met Arg Lys Arg Gly Phe Thr Met
        305                 310                 315

gga ata gga cag tat ata ttc gta aat tgc ccc tcg att tcc ttc ctg 1129
Gly Ile Gly Gln Tyr Ile Phe Val Asn Cys Pro Ser Ile Ser Phe Leu
    320                 325                 330

gaa tgg cat ccc ttt act ctg acc tct gct cca gag gaa gaa ttt ttc 1177
Glu Trp His Pro Phe Thr Leu Thr Ser Ala Pro Glu Glu Glu Phe Phe
335                 340                 345                 350

tcc att cat att cga gca gca ggg gac tgg aca gaa aat ctc ata agg 1225
Ser Ile His Ile Arg Ala Ala Gly Asp Trp Thr Glu Asn Leu Ile Arg
                355                 360                 365

aca ttt gaa caa cag cac tca cca atg ccc agg atc gag gtg gat ggt 1273
Thr Phe Glu Gln Gln His Ser Pro Met Pro Arg Ile Glu Val Asp Gly
            370                 375                 380

ccc ttt ggc aca gtc agt gag gat gtc ttc cag tac gaa gtg gct gta 1321
Pro Phe Gly Thr Val Ser Glu Asp Val Phe Gln Tyr Glu Val Ala Val
        385                 390                 395

ctg gtt ggg gca ggg att ggc gtc act ccc ttt gct tcc ttc ttg aaa 1369
Leu Val Gly Ala Gly Ile Gly Val Thr Pro Phe Ala Ser Phe Leu Lys
    400                 405                 410

tct atc tgg tac aaa ttc cag cgt gca cac aac aag ctg aaa aca caa 1417
Ser Ile Trp Tyr Lys Phe Gln Arg Ala His Asn Lys Leu Lys Thr Gln
415                 420                 425                 430

aag atc tat ttc tac tgg att tgt aga gag acg ggt gcc ttt gcc tgg 1465
Lys Ile Tyr Phe Tyr Trp Ile Cys Arg Glu Thr Gly Ala Phe Ala Trp
                435                 440                 445

ttc aac aac tta ttg aat tcc ctg gaa caa gag atg gac gaa tta ggc 1513
Phe Asn Asn Leu Leu Asn Ser Leu Glu Gln Glu Met Asp Glu Leu Gly
            450                 455                 460

aaa ccg gat ttc cta aac tac cga ctc ttc ctc act ggc tgg gat agc 1561
Lys Pro Asp Phe Leu Asn Tyr Arg Leu Phe Leu Thr Gly Trp Asp Ser
        465                 470                 475

aac att gct ggt cat gca gca tta aac ttt gac aga gcc act gac gtc 1609
Asn Ile Ala Gly His Ala Ala Leu Asn Phe Asp Arg Ala Thr Asp Val
    480                 485                 490

ctg aca ggt ctg aaa cag aaa acc tcc ttt ggg aga cca atg tgg gac 1657
Leu Thr Gly Leu Lys Gln Lys Thr Ser Phe Gly Arg Pro Met Trp Asp
495                 500                 505                 510

aat gag ttt tct aga ata gct act gcc cac ccc aag tct gtg gtg ggg 1705
Asn Glu Phe Ser Arg Ile Ala Thr Ala His Pro Lys Ser Val Val Gly
                515                 520                 525

gtt ttc tta tgc ggc cct ccg act ttg gca aaa agc ctg cgc aaa tgc 1753
Val Phe Leu Cys Gly Pro Pro Thr Leu Ala Lys Ser Leu Arg Lys Cys
            530                 535                 540

tgt cgg cgg tac tca agt ctg gat cct agg aag gtt caa ttc tac ttc 1801
Cys Arg Arg Tyr Ser Ser Leu Asp Pro Arg Lys Val Gln Phe Tyr Phe
        545                 550                 555

aac aaa gaa acg ttc tga attggaggaa gccgcacagt agtacttctc 1849
Asn Lys Glu Thr Phe
    560

catcttcctt ttcactaacg tgtgggtcag ctactagata gtccgttgtc gcacaaggac 1909

ttcactccca tcttaaagtt gactcaactc catcattctt gggctttggc aacatgagag 1969

ctgcataact cacaattgca aaacacatga attattattg gggggattgt aaatccttct 2029

gggaaacctg cctttagctg aatcttgctg gttgacactt gcacaattta acctcaggtg 2089

tcttggttga tacctgataa tcttccctcc cacctgtccc tcacagaaga tttctaagta 2149

gggtgatttt aaaatattta ttgaatccac gacaaaacaa taatcataaa taataaacat 2209

aaaattacca agattcccac tcccatatca tacccactaa gaacatcgtt atacatgagc 2269

ttatcatcca gtgtgaccaa caatttatac tttactgtgc caaaataatc ttcatctttg 2329

cttattgaac aattttgctg actttcccta gtaatatctt aagtatatta actggaatca 2389

aatttgtatt atagttagaa gccaactata ttgccagttt gtattgtttg aaataactgg 2449

aaaggcctga cctacatcgt ggggtaattt aacagaagct ctttccattt tttgttgttg 2509

ttgttaaaga gttttgttta tgaatgtgtt ataaaaagaa aataaaaagt tataattttg 2569

acggaaaa 2577

<210> 4
<211> 563
<212> PRT
<213> Rattus norvegicus

<400> 4

Met Gly Asn Trp Leu Val Asn His Trp Leu Ser Val Leu Phe Leu Val
1               5                   10                  15

Ser Trp Leu Gly Leu Asn Ile Phe Leu Phe Val Tyr Val Phe Leu Asn
            20                  25                  30

Tyr Glu Lys Ser Asp Lys Tyr Tyr Tyr Thr Arg Glu Ile Leu Gly Thr
        35                  40                  45

Ala Leu Ala Leu Ala Arg Ala Ser Ala Leu Cys Leu Asn Phe Asn Ser
    50                  55                  60

Met Val Ile Leu Ile Pro Val Cys Arg Asn Leu Leu Ser Phe Leu Arg
65                  70                  75                  80

Gly Thr Cys Ser Phe Cys Asn His Thr Leu Arg Lys Pro Leu Asp His
                85                  90                  95

Asn Leu Thr Phe His Lys Leu Val Ala Tyr Met Ile Cys Ile Phe Thr
            100                 105                 110

Ala Ile His Ile Ile Ala His Leu Phe Asn Phe Glu Arg Tyr Ser Arg
        115                 120                 125

Ser Gln Gln Ala Met Asp Gly Ser Leu Ala Ser Val Leu Ser Ser Leu
    130                 135                 140

Phe His Pro Glu Lys Glu Asp Ser Trp Leu Asn Pro Ile Gln Ser Pro
145                 150                 155                 160

Asn Val Thr Val Met Tyr Ala Ala Phe Thr Ser Ile Ala Gly Leu Thr
                165                 170                 175

Gly Val Val Ala Thr Val Ala Leu Val Leu Met Val Thr Ser Ala Met
            180                 185                 190

Glu Phe Ile Arg Arg Asn Tyr Phe Glu Leu Phe Trp Tyr Thr His His
        195                 200                 205

Leu Phe Ile Ile Tyr Ile Ile Cys Leu Gly Ile His Gly Leu Gly Gly
    210                 215                 220

Ile Val Arg Gly Gln Thr Glu Glu Ser Met Ser Glu Ser His Pro Arg
225                 230                 235                 240

Asn Cys Ser Tyr Ser Phe His Glu Trp Asp Lys Tyr Glu Arg Ser Cys
                245                 250                 255

Arg Ser Pro His Phe Val Gly Gln Pro Pro Glu Ser Trp Lys Trp Ile
            260                 265                 270

Leu Ala Pro Ile Ala Phe Tyr Ile Phe Glu Arg Ile Leu Arg Phe Tyr
        275                 280                 285

Arg Ser Arg Gln Lys Val Val Ile Thr Lys Val Val Met His Pro Cys
    290                 295                 300

Lys Val Leu Glu Leu Gln Met Arg Lys Arg Gly Phe Thr Met Gly Ile
305                 310                 315                 320

Gly Gln Tyr Ile Phe Val Asn Cys Pro Ser Ile Ser Phe Leu Glu Trp
                325                 330                 335

His Pro Phe Thr Leu Thr Ser Ala Pro Glu Glu Glu Phe Phe Ser Ile
            340                 345                 350

His Ile Arg Ala Ala Gly Asp Trp Thr Glu Asn Leu Ile Arg Thr Phe
        355                 360                 365

Glu Gln Gln His Ser Pro Met Pro Arg Ile Glu Val Asp Gly Pro Phe
    370                 375                 380

Gly Thr Val Ser Glu Asp Val Phe Gln Tyr Glu Val Ala Val Leu Val
385                 390                 395                 400

Gly Ala Gly Ile Gly Val Thr Pro Phe Ala Ser Phe Leu Lys Ser Ile
                405                 410                 415

Trp Tyr Lys Phe Gln Arg Ala His Asn Lys Leu Lys Thr Gln Lys Ile

420                 425                 430

Tyr Phe Tyr Trp Ile Cys Arg Glu Thr Gly Ala Phe Ala Trp Phe Asn
        435                 440                 445

Asn Leu Leu Asn Ser Leu Glu Gln Glu Met Asp Glu Leu Gly Lys Pro
    450                 455                 460

Asp Phe Leu Asn Tyr Arg Leu Phe Leu Thr Gly Trp Asp Ser Asn Ile
465                 470                 475                 480

Ala Gly His Ala Ala Leu Asn Phe Asp Arg Ala Thr Asp Val Leu Thr
                485                 490                 495

Gly Leu Lys Gln Lys Thr Ser Phe Gly Arg Pro Met Trp Asp Asn Glu
            500                 505                 510

Phe Ser Arg Ile Ala Thr Ala His Pro Lys Ser Val Val Gly Val Phe
        515                 520                 525

Leu Cys Gly Pro Pro Thr Leu Ala Lys Ser Leu Arg Lys Cys Cys Arg
    530                 535                 540

Arg Tyr Ser Ser Leu Asp Pro Arg Lys Val Gln Phe Tyr Phe Asn Lys
545                 550                 555                 560

Glu Thr Phe

<210>  5
<211>  20
<212>  DNA
<213>  Artificial sequence

<220>
<223>  Forward primer for Nox1 gene

<400>  5
ggagcaggaa ttggggtcac                                                20

<210>  6
<211>  20
<212>  DNA
<213>  Artificial sequence

<220>
<223>  Reverse primer for Nox1 gene

<400>  6
ttgctgtccc atccggtgag                                                20

<210>  7
<211>  21
<212>  DNA
<213>  Artificial sequence

<220>
<223>  Forward primer for human Nox1 gene

<400>  7
ccactgtagg cgccctaagt t                                                21


<210>  8
<211>  20
<212>  DNA
<213>  Artificial sequence

<220>
<223>  Reverse primer for human Nox1 gene

<400>  8
aagaatgacc ggtgcaagga                                                  20


<210>  9
<211>  25
<212>  DNA
<213>  Artificial sequence

<220>
<223>  TaqMan probe

<400>  9
aagggcatcc ccctgagtct tggaa                                            25


<210>  10
<211>  58
<212>  DNA
<213>  Artificial sequence

<220>
<223>  siRNA for human Nox1 gene

<400>  10
gcgtggcttc agcatggaat tcaagagatt ccatgctgaa gccacgcttt tttggaaa        58


<210>  11
<211>  58
<212>  DNA
<213>  Artificial sequence

<220>
<223>  siRNA for human Nox1 gene

<400>  11
gggctttcga acaacaatat tcaagagata ttgttgttcg aaagcccttt tttggaaa        58

<210> 12
<211> 59
<212> DNA
<213> Artificial sequence

<220>
<223> siRNA for rat Nox1 gene

<400> 12
gttatgagaa gtctgacaag ttcaagagac ttgtcagact tctcataatt ttttggaaa 59


<210> 13
<211> 58
<212> DNA
<213> Artificial sequence

<220>
<223> siRNA for rat Nox1 gene

<400> 13
gattcttggc taaatcccat tcaagagatg ggatttagcc aagaatcttt tttggaaa 58


<210> 14
<211> 58
<212> DNA
<213> Artificial sequence

<220>
<223> siRNA for rat Nox1 gene

<400> 14
ggacatttga acaacagcat tcaagagatg ctgttgttca aatgtccttt tttggaaa 58


<210> 15
<211> 20
<212> DNA
<213> Artificial sequence

<220>
<223> Forward primer for Nox1 gene

<400> 15
ggtcactccc tttgcttcca 20


<210> 16
<211> 21
<212> DNA
<213> Artificial sequence

<220>
<223> Reverse primer for rat Nox1 gene

<400> 16
ggcaaaggca cctgtctctc t 21

<210>  17
<211>  20
<212>  DNA
<213>  Artificial sequence

<220>
<223>  TaqManMGB probe

<400>  17
tccagtagaa atagatcttt                                                    20


<210>  18
<211>  65
<212>  DNA
<213>  Artificial sequence

<220>
<223>  siRNA construction

<400>  18
gatcccgtta tgagaagtct gacaagttca agagacttgt cagacttctc ataatttttt      60

ggaaa                                                                    65


<210>  19
<211>  65
<212>  DNA
<213>  Artificial sequence

<220>
<223>  siRNA construction

<400>  19
agcttttcca aaaaattatg agaagtctga caagtctctt gaacttgtca gacttctcat      60

aacgg                                                                    65


<210>  20
<211>  64
<212>  DNA
<213>  Artificial sequence

<220>
<223>  siRNA construction

<400>  20
gatcccgatt cttggctaaa tcccattcaa gagatgggat ttagccaaga atcttttttg      60

gaaa                                                                     64


<210>  21
<211>  64
<212>  DNA
<213>  Artificial sequence

<220>
<223>  siRNA construction

<400>  21
agcttttcca aaaaagattc ttggctaaat cccatctctt gaatgggatt tagccaagaa      60

tcgg                                                                    64


<210>  22
<211>  64
<212>  DNA
<213>  Artificial sequence

<220>
<223>  siRNA construction

<400>  22
gatcccggac atttgaacaa cagcattcaa gagatgctgt tgttcaaatg tccttttttg      60

gaaa                                                                    64


<210>  23
<211>  64
<212>  DNA
<213>  Artificial sequence

<220>
<223>  siRNA construction

<400>  23
agcttttcca aaaaaggaca tttgaacaac agcatctctt gaatgctgtt gttcaaatgt      60

ccgg                                                                    64


<210>  24
<211>  17
<212>  DNA
<213>  Artificial sequence

<220>
<223>  M13 primer

<400>  24
gttttcccag tcacgac                                                      17


<210>  25
<211>  21
<212>  DNA
<213>  Artificial sequence

<220>
<223>  3.0 Rev primer

<400>  25
gagttagctc actcattagg c                                                 21


<210>  26
<211>  19

<212>  DNA
<213>  Artificial sequence

<220>
<223>  Forward primer for Nox1 gene

<400>  26
atgggaaact ggctggtta                                                    19


<210>  27
<211>  21
<212>  DNA
<213>  Artificial sequence

<220>
<223>  Reverse primer for Nox1 gene

<400>  27
tcagaacgtt tctttgttga a                                                 21